(19) Europäisches Patentamt
European Patent Office
Office européen des brevets

(11) Veröffentlichungsnummer: **0 247 483**
**A2**

(12) **EUROPÄISCHE PATENTANMELDUNG**

(21) Anmeldenummer: **87107162.7**

(22) Anmeldetag: **18.05.87**

(51) Int. Cl.⁴: **C 07 C 125/065**, C 07 C 125/073, C 07 C 125/077

(30) Priorität: **28.05.86 DE 3618007**

(43) Veröffentlichungstag der Anmeldung: **02.12.87**
**Patentblatt 87/49**

(84) Benannte Vertragsstaaten: **DE FR GB IT**

(71) Anmelder: **BAYER AG, Konzernverwaltung RP**
**Patentabteilung, D-5090 Leverkusen 1 Bayerwerk (DE)**

(72) Erfinder: **Stammann, Günter, Dr., Korweg 9,**
**D-5270 Gummersbach 1 (DE)**
Erfinder: **Bottenbruch, Ludwig, Dr., Wöhlerstrasse 5,**
**D-4150 Krefeld (DE)**
Erfinder: **Waldmann, Helmut, Dr.,**
**Henry-T.-v.-Böttinger-Strasse 15,**
**D-5090 Leverkusen 1 (DE)**

(54) **Verfahren zur Herstellung von Hydroxyphenylurethanen.**

(57) Hydroxyphenylurethane der Formel (3)

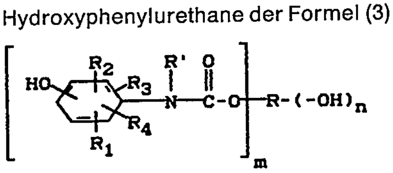

(3),

in der die verwendeten Symbole die in der Beschreibung angegebene Bedeutung haben, werden hergestellt, indem man Verbindungen mit mindestens einer harnstoff- oder urethanartig eingebundenen Struktureinheit der Formel (1)

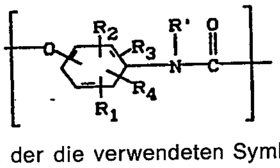

(1),

in der die verwendeten Symbole die in der Beschreibung angegebene Bedeutung haben, mit Alkoholen der Formel (2)

$$(HO-)_m-R-(-OH)_n \qquad (2),$$

in der die verwendeten Symbole die in der Beschreibung angegebene Bedeutung haben, bei erhöhter Temperatur und gegebenenfalls in Gegenwart von Lösungsmitteln und/oder Katalysatoren umsetzt.

EP 0 247 483 A2

0 247 483

BAYER AKTIENGESELLSCHAFT
Konzernverwaltung RP
Patentabteilung

5090 Leverkusen, Bayerwerk

Gai/Ke-c

## Verfahren zur Herstellung von Hydroxyphenylurethanen

Die vorliegende Erfindung betrifft ein Verfahren zur Herstellung von Hydroxyphenylurethanen durch eine Alkoholysereaktion mit höherwertigen Alkoholen.

Aus der DE-OS 2 514 540 ist u.a. bekannt, daß man Hydroxyphenylurethane der Formel

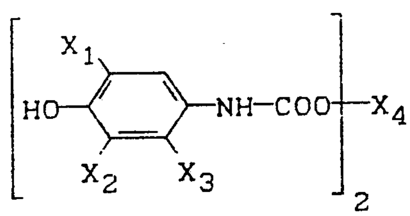

in der

$X_1$ $C_3$-$C_4$-Alkyl,

$X_2$ $C_1$-$C_4$-Alkyl,

$X_3$ Wasserstoff oder Methyl und

$X_4$ $C_2$-$C_8$-Alkylen, Phenylen oder einen Rest der Formel

$$-(CH_2)_{2-3}-X-(CH_2)_{2-3}$$

mit X = Sauerstoff oder Schwefel

bedeuten können, herstellen kann, indem man ein entsprechendes p-Aminophenol mit einem Chlorkohlensäureester umsetzt oder ein substituiertes p-Hydroxyphenylisocyanat mit einem Alkohol umsetzt. Ähnliche Methoden zur Herstellung von einfachen Hydroxyphenylalkylisocyanaten sind beispielsweise in der DE-OS 1 643 763 und in der US-PS 933 470 beschrieben.

Hydroxyphenylalkylurethane können auch durch direkte Carbonylierung von Nitrophenolen in Gegenwart von Alkoholen unter Verwendung spezieller Katalysatorsysteme hergestellt werden, wie es beispielsweise in der EP-OS 94 861, der EP-OS 86 018 und in der DE-OS 3 406 230 beschrieben ist. Alle diese Umsetzungen gelingen im allgemeinen nur mit einwertigen Alkoholen und kleinen Alkylresten in zufriedenstellender Weise.

Die Herstellung von Urethanen durch umesterungsartige Reaktionen ist bekannt und wird beispielsweise in der BE-PS 861 700, sowie von H. Hagemann (Hrsg.) in "Methoden der organischen Chemie" Bd. E4, Stuttgart 1983, S. 168-169 und von Houben-Weyl (Hrsg.) "Methoden der organischen Chemie"

Bd. VIII/3, Stuttgart 1952, S. 146 und in den dort angeführten Zitaten beschrieben. Die Herstellung von Hydroxyphenylurethanen, insbesondere solchen, die mehr als eine Hydroxylgruppe und gegebenenfalls auch nicht-phenolische Hydroxylgruppen aufweisen durch umesterungsartige Reaktionen ist bisher nicht bekannt.

Es wurde nun überraschenderweise ein Verfahren zur Herstellung von Hydroxyphenylurethanen der Formel (3) gefunden

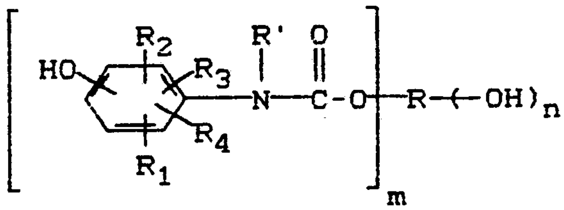

(3),

in der

m für eine ganze Zahl von 1 bis 4 und

n für Null oder eine ganze Zahl von 1 bis 3 steht und die Summe von m + n mindestens 2 und höchstens 4 beträgt,

$R_1$ bis $R_4$ unabhängig voneinander Wasserstoff, Halogen, Cyano, Alkyl, Cycloalkyl, Aryl, Aralkyl, Oxyalkyl, Oxyphenyl oder Oxybenzyl,

R' Wasserstoff, Alkyl, Acyl oder Phenyl und

R im Falle von m + n von 2 bis 4

a) einen m + n-bindigen, geradkettigen, verzweigten oder cyclischen, gesättigten oder ungesättigten, gegebenenfalls substituierten Kohlenwasserstoffrest mit insgesamt 2 bis 30 C-Atomen oder

b) einen Rest der Formel (2.1)

$$Z{-}\left[\left(-OC_2H_3Y-\right)_r\right]_{m+n} \qquad (2.1)$$

mit Z = einem geradkettigen, verzweigten oder cyclischen, gesättigten oder ungesättigten, gegebenenfalls substituierten Rest mit insgesamt 3 bis 20 C-Atomen,

Y = H und/oder $CH_3$ (unabhängig von benachbarten Kettengliedern) und

r = einer ganzen Zahl von 1 bis 1000 oder einem gegebenenfalls nicht ganzzahligen Mittelwert von 1 bis 1000 und

im Falle von m + n = 2 auch

c) einen Rest der Formel (2.2)

$$-\!\left[(CH_2)_s-O\right]_t(CH_2)_s- \qquad (2.2)$$

mit s = einer ganzen Zahl von 3 bis 6 und dem Produkt s · t = einer ganzen Zahl von 6 bis 3000 oder oder einem gegebenenfalls nicht ganzzahligen Mittelwert von 6 bis 3000 oder

d) einen Rest der Formel (2.3)

$$-(C_2H_3Y-O)_u-(C_2H_3Y)- \qquad (2.3)$$

mit u = einer ganzen Zahl von 2 bis 200 000 oder einem gegebenenfalls nicht ganzzahligen Mittelwert von 2 bis 200 000 und

Y in der gleichen Bedeutung wie bei Formel (2.1),

bedeuten,

das dadurch gekennzeichnet ist, daß man Verbindungen mit mindestens einer harnstoff- oder urethanartig eingebundenen Struktureinheit der Formel (1)

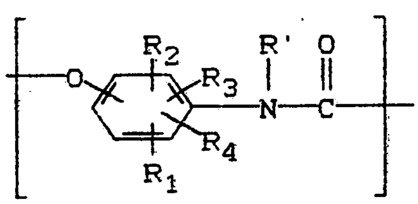

(1),

in der

$R_1$ bis $R_4$ und R' die bei Formel (3) angegebene Bedeutung haben,

mit Alkoholen der Formel (2)

$$(HO \!\!-\!\!)_m R (\!\!-\!\! OH)_n \qquad (2),$$

in der

m, n und R die bei Formel (3) angegebene Bedeutung haben, bei erhöhter Temperatur und gegebenenfalls in Gegenwart von Lösungsmitteln und/oder Katalysatoren umsetzt.

Vorzugsweise werden mit dem erfindungsgemäßen Verfahren Hydroxyphenylurethane hergestellt, bei welchen in Formel (3) R im Falle a) 6 bis 30 C-Atome enthält, im Falle b) r für 1 bis 100 steht, im Falle c) das Produkt s · t für 6 bis 30 steht und im Falle d) u für eine Zahl von 2 bis 250 steht und weiterhin werden mit den erfindungsgemäßen Verfahren vorzugsweise solche Hydroxyphenylurethane hergestellt, die nicht in der DE-OS 2 514 540 beschrieben sind.

Bei den als Einsatzstoffe in das erfindungsgemäße Verfahren benötigten Verbindungen mit mindestens einer harnstoff- oder urethanartig eingebundenen Struktureinheit der Formel (1) handelt es sich bevorzugt um Verbindungen der Formeln (4) bis (7).

Bei Formel (4)

$$-(-1-)_q \qquad (4),$$

in der

1 für eine Struktureinheit der Formel (1) und

q für 500 bis $10^6$ dividiert durch das Molekulargewicht der jeweiligen Struktureinheit (1)

stehen, handelt es sich um Polyurethane. Die Endgruppen solcher Polyurethane sind für das erfindungsgemäße Verfahren unwesentlich, sie ergeben sich zwangsweise aus den Herstellungsverfahren für die Polyurethane. Bei bevorzugten Polyurethanen der Formel (4) enthält höchstens einer der Reste $R_1$ bis $R_4$, die in (1) enthalten sind, mehr als 2 C-Atome.

Bei Formel (5)

$$H-(-1-)-OR_5 \qquad (5),$$

in der

1 für eine Struktureinheit der Formel (1) und

$R_5$ für gegebenenfalls substituiertes $C_1$-$C_5$-Alkyl, Phenyl oder Benzyl

stehen, handelt es sich um Hydroxyphenylurethane. Als Substituenten für $R_5$ kommen z.B. in Frage: Hydroxyl, Halogen und/oder Alkoxygruppen.

Bei Formel (6)

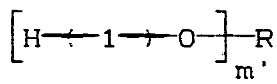 (6),

in der

1     für eine Struktureinheit der Formel (1) und

m'    für eine ganze Zahl von 2 bis 4 stehen und

R     die bei Formel (3) angegebene Bedeutung hat,

handelt es sich ebenfalls um Hydroxyphenylurethane. Die Hydroxyphenylurethane der Formel (6) werden insbesondere zur Herstellung von Hydroxyphenylurethanen der Formel (3) eingesetzt, bei denen n von Null verschieden ist und vorzugsweise für 1 steht.

Bei Formel (7)

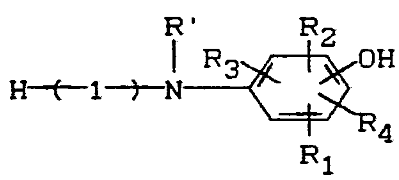 (7),

in der

1     für eine Struktureinheit der Formel (1) und

$R_1$ bis $R_4$ und R' die bei Formel (3) angegebene Bedeutung haben, handelt es sich um symmetrisch substituierte Harnstoffe.

Auch andere harnstoff- oder urethanartig eingebundene Struktureinheiten der Formel (1) enthaltende Verbindungen, beispielsweise solche vom Typ der Allophanate und Biurete, sind als Einsatzstoffe für das erfindungsgemäße Verfahren geeignet. Vom praktischen Interesse sind solche Verbindungen meist nur dann, wenn sie als Nebenprodukte bei der Herstellung von Verbindungen der Formeln (4) bis (7) anfallen. Sie können dann, vorzugsweise im Gemisch mit Verbindungen der Formeln (4) bis (7), in das erfindungsgemäße Verfahren eingesetzt werden.

Für das erfindungsgemäße Verfahren geeignete Alkohole der Formel (2) sind beispielsweise Ethylenglykol, 1,3-Propylenglykol, 1,4-Butandiol, 1,3-Butandiol, 2-Methyl-1,3-propandiol, 2,3-Butandiol, 1,5-Pentandiol, 2,2-Dimethyl-1,3-propandiol, Diethylenglykol, 2-Hydroxymethyl-2-methyl-1,3-propandiol, OH-terminierte, polymere Tetrahydrofurane und die isomeren Dipropylenglykole. In Formel (2) bezeichnet der Index n jeweils die Anzahl derjenigen Hydroxylgruppen, die gegebenenfalls als alkoholische Hydroxylgruppen in den Hydroxyphenylurethanen der Formel (3) verbleiben, während der Index m denjenigen Teil der Hydroxylgruppen bezeichnet, die in Hydroxyphenylurethangruppen überführt werden. Bevorzugte Alkohole der Formel (2) sind weiter unten genannt.

Zum Einsatz in das erfindungsgemäße Verfahren sind auch solche Alkohole der Formel (2) geeignet, bei denen R in der Bedeutung von a) als Substituenten beispielsweise Halogenatome, vorzugsweise Fluor-oder Chloratome, Alkoxy-

gruppen, vorzugsweise $C_1$- bis $C_6$-Alkoxygruppen und/oder Estergruppen, vorzugsweise solche mit 2 bis 5 C-Atomen, enthält. Bei ungesättigten Resten kann es sich beispielsweise um solche handeln, die 1 bis 3 ethylenische Doppelbindungen oder 1 bis 3 aromatische Ringe enthalten.

Vorzugsweise handelt es sich bei R in Formel (2) in der Bedeutung von a) um eine gesättigte Kohlenwasserstoffkette mit insgesamt 6 bis 20 C-Atomen, die gegebenenfalls 1 bis 3 gesättigte Kohlenwasserstoffseitenketten mit je 1 bis 3 C-Atomen, gegebenenfalls 1 bis 8 Fluor- und/oder Chloratomen, gegebenenfalls 1 bis 3 Alkoxygruppen mit je 2 bis 4 C-Atomen, gegebenenfalls 1 bis 2 Estergruppen mit je 2 bis 4 C-Atomen, gegebenenfalls 1 bis 3 Cycloalkylreste und/oder gegebenenfalls 1 bis 3 Phenylreste als Substituenten enthält, sowie um Kohlenwasserstoffreste der gleichen Art, bei denen das Kohlenwasserstoffgerüst 1 bis 2 ethylenische Doppelbindungen enthält.

Beispiele für Reste R in der Bedeutung von a) sind folgende: α,ω-n-Alkylen mit 6 bis 30 C-Atomen, 2,5-Hexandiyl, 4-Methyl-1,4-pentandiyl, 3-Methyl-1,5-pentandiyl, 2-Methyl-2-(1-propyl)-1,3-propandiyl, 2,2-Diethyl-1,3-propandiyl, 2-Ethyl-1,3-hexandiyl, 2,5-Dimethyl-1,5-hexandiyl, 2,2,4-Trimethyl-1,3-pentandiyl, 2,2,4-Trimethyl-1,6-hexandiyl, 1,12-Octadecandiyl, 2-(1-Propoxy)-1,3-propandiyl, 2-(2-Propoxymethyl)-2-ethyl-1,3-propandiyl, 2,3-Dichlorbutandiyl, Perfluorhexan-1,6-diyl, 2-Acetoxymethyl-2-methyl-1,3-propandiyl, Hexadec-8-en-1,16-diyl, 15-Methylhexadec-2-en-1,15-diyl, 2-Cyclohexyliden-1,3-propandiyl

und 2,2-Diphenyl-1,3-propandiyl. Von diesen Resten sind die bis einschließlich 1,12-Octadecandiyl genannten bevorzugt, ganz besonders bevorzugt α,ω-n-Alkylen mit 8 bis 20 C-Atomen.

R in der Bedeutung von a) kann von Einzelmolekül zu Einzelmolekül variieren. Insbesondere können auch Alkohole der Formel (2) mit R in der Bedeutung von a) als Gemische vorliegen, bei denen verschiedene R isomer zueinander sind und/oder einer homologen Reihe angehören.

R in Formel (2) in der Bedeutung von b) (siehe Formel (2.1)) kann als Strukturteil Z beispielsweise einen Rest enthalten, wie er zuvor für R in der Bedeutung von a) beschrieben wurde, jedoch von 3 C-Atomen an und nur solche bis 20 C-Atomen. Z kann auch Heteroatome enthalten, beispielsweise bis zu 4 ausgewählt aus Phosphor, Stickstoff, Schwefel und/oder Silicium oder bis zu 12 ausgewählt aus Sauerstoff und/oder Chlor. Weiterhin kann Z der Formel (2.4)

$$-(CH_2)_v-O-[(CH_2)_v-O]_w-(CH_2)_v- \qquad (2.4)$$

entsprechen,

in der

v für eine ganze Zahl von 3 bis 6 und

w für Null oder 1 stehen.

Bei Z kann es sich auch um Mischungen verschiedener Reste z.B. verschiedener oligomerer Reste der Formel (2.4) handeln. Z kann sich auch von einem gesättigten cyclischen oder aromatischen Grundgerüst ableiten.

R in Formel (2) in der Bedeutung von b) (siehe Formel (2.1)) kann als Strukturteil Z beispielsweise enthalten: α,ω-n-Alkylen mit 3 bis 20 C-Atomen, 2,5-Hexandiyl, 4-Methyl-1,4-pentandiyl, 3-Methyl-1,5-pentandiyl, 2-Methyl-2-(1-propyl)-1,3-propandiyl, 2,2-Diethyl-1,3-propandiyl, 2-Ethyl-1,3-hexandiyl, 2,5-Dimethyl-1,5-hexandiyl, 2,2,4-Trimethyl-1,3-pentandiyl, 2,2,4-Trimethyl-1,6-hexandiyl, 1,12-Octadecandiyl, 2-(1-Propoxy)-1,3-propandiyl, 2-(2-Propoxymethyl)-2-ethyl-1,3-propandiyl, 2,3-Dichlorbutandiyl, Perfluorhexan-1,6-diyl, 2-Acetoxymethyl-2-methyl-1,3-propandiyl, Hexadec-8-en-1,16-diyl, 15-Methylhexadec-2-en-1,15-diyl, 2-Cyclohexyliden-1,3-propandiyl und 2,2-Diphenyl-1,3-propandiyl, Reste der Formel (2.4) mit v = 3 und w = 0, v = 3 und w = 1, v = 4 und w = 0, v = 4 und w = 1, v = 5 und w = 0, v = 5 und w = 1, v = 6 und w = 0 und v = 6 und w = 1 und Reste der Formeln (8.1) bis (14.10).

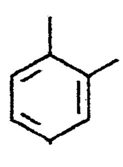
(8.1)

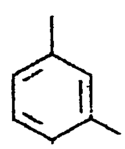
(8.2)

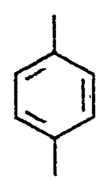
(8.3)

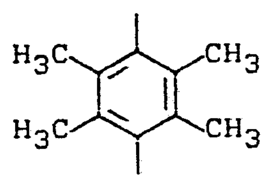
(8.4)

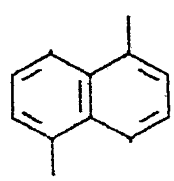
(8.5)

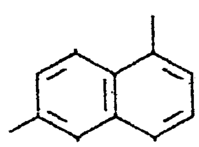
(8.6)

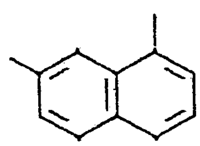
(8.7)

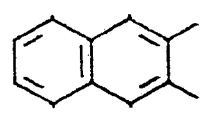
(8.8)

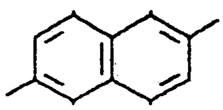

(8.9)

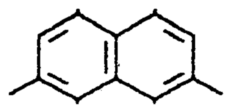

(8.10)

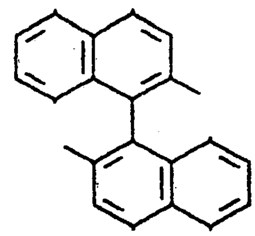

(8.11)

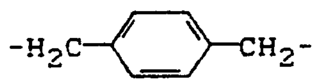

(9.1)

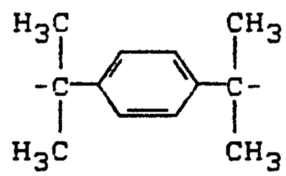

(9.2)

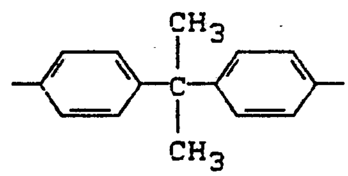

(9.3)

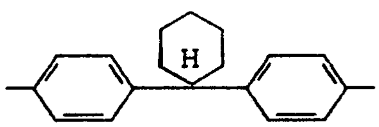

(9.4)

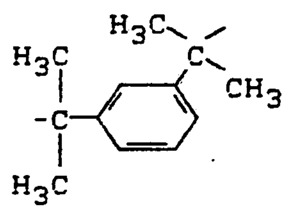

(9.5)

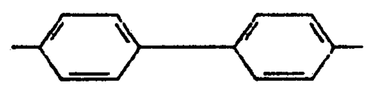

(9.6)

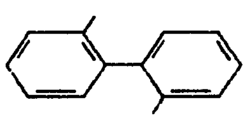

(9.7)

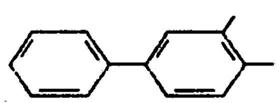

(9.8)

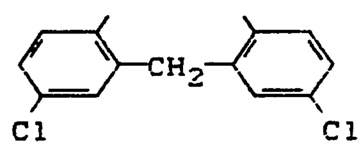

(9.9)

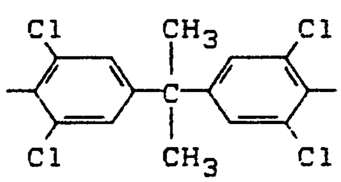

(9.10)

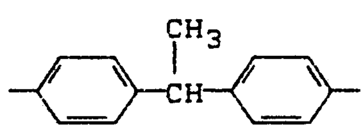

(9.11)

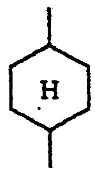

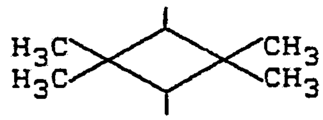

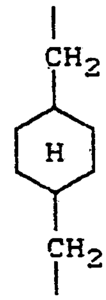

(10.1) (10.2) (10.3)

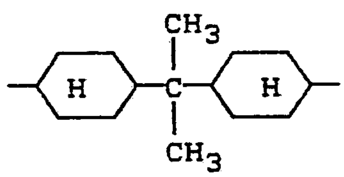

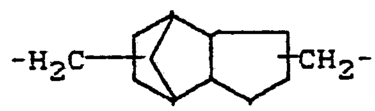

(10.4) (10.5)

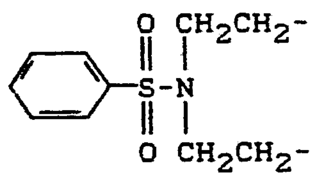

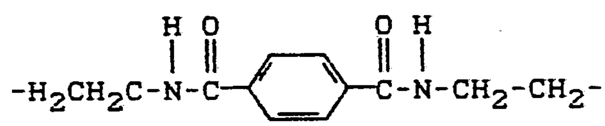

(11.1) (11.2)

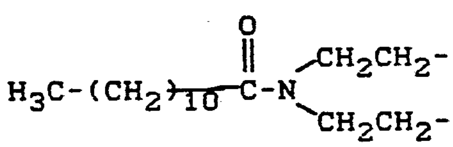

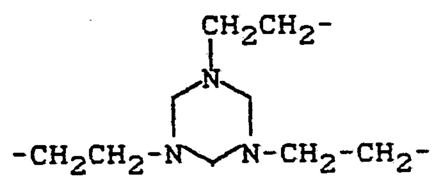

(11.3) (11.4)

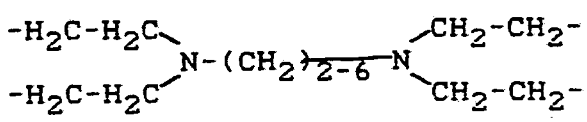

(11.5)

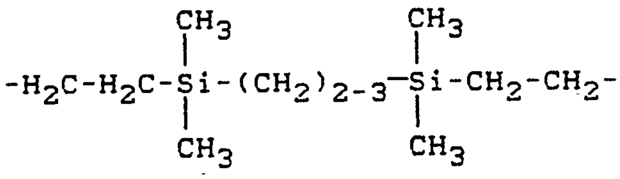

(12.1)

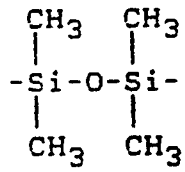

(12.2)

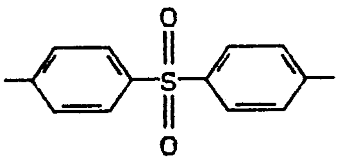

(13.1)

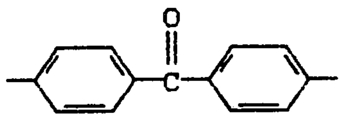

(13.2)

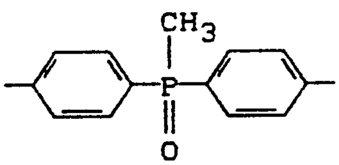

(13.3)

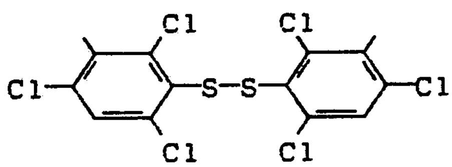

(13.4)

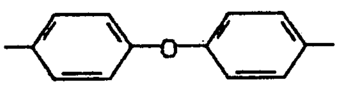

(13.5)

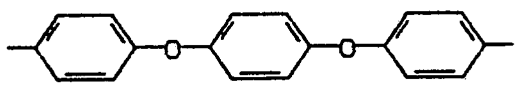

(13.6)

$-CH_2CH_2-S-CH_2-CH_2-$

(13.7)

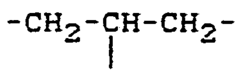

(14.1)

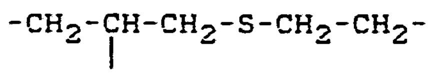

(14.2)

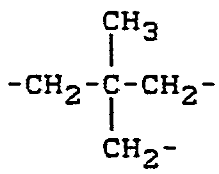

(14.3)

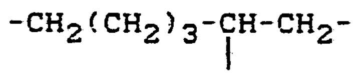

(14.4)

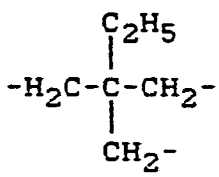

(14.5)

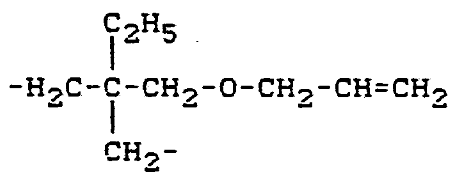

(14.6)

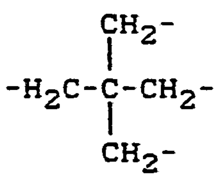

(14.7)

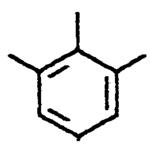

(14.8)

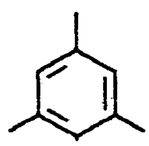

(14.9)

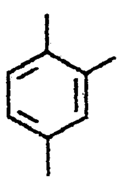

(14.10)

Weiterhin kommen als Z Reste in Frage, die sich durch den Wegfall der alkoholischen OH-Gruppen von solchen Diolen oder Polyolen ableiten, die durch Ethoxylierung und/oder Propoxylierung acider Verbindungen zugänglich sind. Beispiele für solche Diole und Polyole sind aufgeführt in Encyclopedia of Polymer Science and Technology, Vol. 6, S. 103 - 209 (New York 1967) unter dem Stichwort 1,2-Epoxy Polymers, in N. Schönfeldt, Grenzflächenaktive Ethylenoxidaddukte (Stuttgart 1976), S. 13 - 102 und 897 - 986, sowie in N. Schönfeldt, Grenzflächenaktive Ethylenoxidaddukte (Stuttgart 1984), S. 14 - 44 und 421 - 472.

R in der Bedeutung von b) (siehe Formel (2.1)) enthält als Strukturteil Z vorzugsweise einen Rest der Formeln (8.1) bis (10.5) oder (13.1) bis (14.10). In Formel (2.1) steht weiterhin Y vorzugsweise für Wasserstoff und r vorzugsweise für eine ganze Zahl von 1 bis 100, besonders bevorzugt für 1 oder 2.

R in Formel (2) in der Bedeutung von c) (siehe auch Formel (2.2)) steht vorzugsweise für keinen Rest, bei dem m = 2, n = 0, $R_1$ und $R_2$ = $C_3$-oder $C_4$-Alkyl und s · t = 6 ist. Von den Resten der Formel (2.2) sind solche bevorzugt, die den Formeln (15) bis (18) entsprechen.

$$-(CH_2)_3-O\left[(CH_2)_3-O\right]_{x_1}-(CH_2)_3- \quad , x_1 = 1 \text{ bis } 8 \qquad (15)$$

$$-(CH_2)_4-O\left[(CH_2)_4-O\right]_{x_2}-(CH_2)_4- \quad , x_2 = 1 \text{ bis } 5 \qquad (16)$$

$$-(CH_2)_5-O\left[(CH_2)_5-O\right]_{x_3}(CH_2)_5- \quad , x_3 = 1 \text{ bis } 4 \quad (17)$$

$$-(CH_2)_6-O\left[(CH_2)_6-O\right]_{x_4}(CH_2)_6- \quad , x_4 = 1 \text{ bis } 3 \quad (18)$$

Vorzugsweise stehen $x_1$ und $x_4$ in den Formeln (15) bis (18) für 1 oder 2. Reste der Formeln (2.2), insbesondere solche der Formeln (15) bis (18), können auch als Gemisch oligomerer Reste unterschiedlicher Kettenlängen vorliegen. Ferner können Reste der Formel (2.2) innerhalb einer Kette Segmente mit verschiedenen Werten für s aufweisen.

R in der Bedeutung von d) (siehe Formel (2.3)) kann für Reste stehen, die sich von solchen Polyethylenglykolen, Poly-1,2-propylenglykolen oder Mischungen davon, die in beliebiger Weise aus Ethylenoxid und/oder Propylenoxid hergestellt werden können, durch den Wegfall der alkoholischen OH-Gruppen ableiten. Beispielsweise sind solche Herstellverfahren für Polyethylen- und/oder Poly-1,2-propylen-glykole beschrieben in Encyclopedia of Polymer Science and Technology loc. cit. und Ullmanns Enzyklopädie der Technischen Chemie, Band XIX, Seite 31 bis 38 (Weinheim 1980). Vorzugsweise haben Reste der Formel (2.3) ein Molekulargewicht bis zu 11 000, besonders bevorzugt bis zu 3 000. Ferner sind Mischungen von Resten der Formel (2.3) bevorzugt, die eine sehr enge Molekulargewichtsverteilung aufweisen, beispielsweise eine Uneinheitlichkeit von $< 0{,}1$ (Uneinheitlichkeit = $\frac{M_w}{M_n} -1$).

Mit dem erfindungsgemäßen Verfahren werden bevorzugt folgende Hydroxyphenylurethane der Formel (3) hergestellt:

1,5-Bis-(4-hydroxyphenylcarbamoyloxy)-3-methylpentan,
α,ω-Bis-(4-hydroxyphenylcarbamoyloxy)-n-alkan mit $C_6$- bis $C_{30}$-n-alkan, vorzugsweise $C_8$- bis $C_{20}$-n-alkan,
2,5-Bis-(4-hydroxyphenylcarbamoyloxy)-hexan,
1,3-Bis-(4-hydroxyphenylcarbamoyloxy)-2-methyl-2-(1-propyl-propan,
1,3-Bis-(4-hydroxyphenylcarbamoyloxy)-2,2-diethylpropan,
1,3-Bis-(4-hydroxyphenylcarbamoyloxy)-2-ethylhexan,
1,3-Bis-(4-hydroxyphenylcarbamoyloxy)-2,2,4-trimethylpentan,
1,6-Bis-(4-hydroxyphenylcarbamoyloxy)-2,2,4-trimethylhexan,
1,12-Bis-(4-hydroxyphenylcarbamoyloxy)-octadecan,
1,3-bis-(4-hydroxyphenylcarbamoyloxy)-2-(1-propoxy)-propan,
1,6-Bis-(4-hydroxyphenylcarbamoyloxy)-perfluorhexan,
1,16-Bis-(4-hydroxyphenylcarbamoyloxy)-8-hexadecen,
α,ω-Bis-(4-hydroxy-3,5-dimethyl-phenyl-carbamoyloxy)-n-alkan mit $C_6$- bis $C_{30}$-n-alkan, vorzugsweise mit $C_8$- bis $C_{20}$-n-alkan,
1,12-Bis-(4-hydroxy-3,5-dimethyl-phenyl-carbamoyloxy)-octadecan,
1,12-Bis-(3,5-di-tert.-butyl-4-hydroxyphenylcarbamoyloxy)-octadecan,

α,ω-Bis-(4-hydroxy-3,5-dimethyl-phenyl-carbamoyloxy)-n-alkan mit $C_6$- bis $C_{30}$-n-alkan, vorzugsweise mit $C_8$- bis $C_{20}$-n-alkan,
α,ω-Bis-(3,5-dichlor-4-hydroxyphenyl-carbamoyloxy)-n-alkan mit $C_6$- bis $C_{30}$-n-alkan, vorzugsweise mit $C_8$- bis $C_{20}$-n-alkan,
α,ω-Bis-(3,5-di-tert.-butyl-4-hydroxy-phenylcarbamoyl)-n-alkan mit $C_{10}$- bis $C_{30}$-n-alkan, vorzugsweise mit $C_{10}$- bis $C_{20}$-n-alkan,
1,12-Bis-(3,5-dichlor-4-hydroxyphenyl-carbamoyloxy)-octadecan,
1,12-Bis-(2,3,5,6-tetrachlor-4-hydroxy-phenylcarbamoyloxy)-octadecan,
1,12-Bis-(3-hydroxyphenylcarbamoyloxy)-octadecan,
α,ω-Bis-(3-hydroxyphenylcarbamoyloxy)-n-alkan mit $C_6$- bis $C_{30}$-n-alkan, vorzugsweise mit $C_8$- bis $C_{20}$-n-alkan,
α,ω-Bis-(4-chlor-3-hydroxyphenyl-carbamoyloxy)-n-alkan mit $C_6$- bis $C_{30}$-n-alkan, vorzugsweise mit $C_8$- bis $C_{20}$-n-alkan,
α,ω-Bis-(2,4-dichlor-3-hydroxyphenyl-carbamoyloxy)-n-alkan mit $C_6$- bis $C_{30}$-n-alkan, vorzugsweise mit $C_8$- bis $C_{20}$-n-alkan,
1,9-Bis-(4-hydroxyphenylcarbamoyloxy)-5-oxanonan,
1,13-Bis-(4-hydroxyphenylcarbamoyloxy)-5,9-dioxanonan,
1,7-Bis-(4-hydroxyphenylcarbamoyloxy)-4-oxaheptan,
1,11-Bis-(4-hydroxyphenylcarbamoyloxy)-4,7-dioxaundecan,
1,11-bis-(3,5-di-tert.-butyl-4-hydroxyphenyl-carbamoyloxy)-4,7-dioxaundecan,
1,9-bis-(3-hydroxyphenylcarbamoyloxy)-5-oxanonan,
1-(4-Hydroxyphenylcarbamoyloxy)-5-hydroxy-3-methylpentan,

α-(4-Hydroxyphenylcarbamoyloxy)-ω-hydroxy-n-alkan mit $C_6$- bis $C_{30}$-n-alkan, vorzugsweise mit $C_6$- bis $C_{20}$-n-alkan,
1-(4-Hydroxyphenylcarbamoyloxy)-2,2-diethyl-3-hydroxypropan,
1-(4-Hydroxyphenylcarbamoyloxy)-3-hydroxy-2-methyl-2-(1-propyl)-propan,
1-(4-Hydroxyphenylcarbamoyloxy)-2-ethyl-3-hydroxyhexan,
6-(4-Hydroxyphenylcarbamoyloxy)-1-hydroxy-2,2,4-trimethyl-hexan,
1-(4-Hydroxyphenylcarbamoyloxy)-12-hydroxy-octadecan,
6-(4-Hydroxyphenylcarbamoyloxy)-1,2-dihydroxyhexan,
1-(4-Hydroxyphenylcarbamoyloxy)-3-hydroxy-2,2-bis-(hydroxymethyl)-propan,
1-(4-Hydroxyphenylcarbamoyloxy)-6-hydroxyperfluorhexan,
α-(3,5-Di-tert.-butyl-4-hydroxyphenylcarbamoyloxy)-ω-hydroxy-n-alkan mit $C_6$- bis $C_{30}$-n-alkan, vorzugsweise mit $C_6$- bis $C_{20}$-n-alkan,
1-(3,5-Di-tert.-butyl-4-hydroxyphenylcarbamoyloxy)-12-hydroxyoctadecan,
α-(3,5-Dichlor-4-hydroxyphenylcarbamoyloxy)-ω-hydroxy-n-alkan mit $C_6$- bis $C_{30}$-n-alkan, vorzugsweise mit $C_6$- bis $C_{20}$-n-alkan,
1-(3,5-Dichlor-4-hydroxyphenylcarbamoyloxy)-12-hydroxy-octadecan,
α-(3-Hydroxyphenylcarbamoyloxy)-ω-hydroxy-n-alkan mit $C_6$- bis $C_{30}$-n-alkan, vorzugsweise mit $C_6$- bis $C_{20}$-n-alkan,
1-(3-Hydroxyphenylcarbamoyloxy)-12-hydroxyoctadecan,
1-(4-Hydroxyphenylcarbamoyloxy)-7-hydroxy-4-oxa-heptan,
1-(4-Hydroxyphenylcarbamoyloxy)-9-hydroxy-5-oxanonan und
1-(3,5-Di-tert.-butyl-4-hydroxyphenyl-carbamoyloxy)-9-hydroxy-5-oxanonan,

sowie solche der Formeln (19) bis (47):

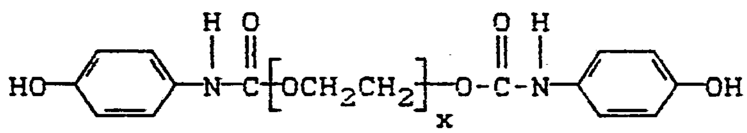

(19)

x = 3 bis 220, vorzugsweise 3 bis 10.

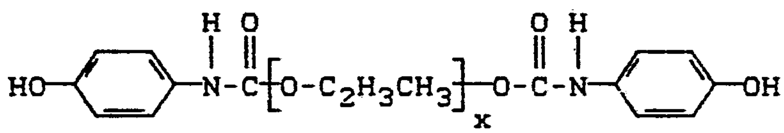

(20)

x = 3 bis 110, vorzugsweise 3 bis 10.

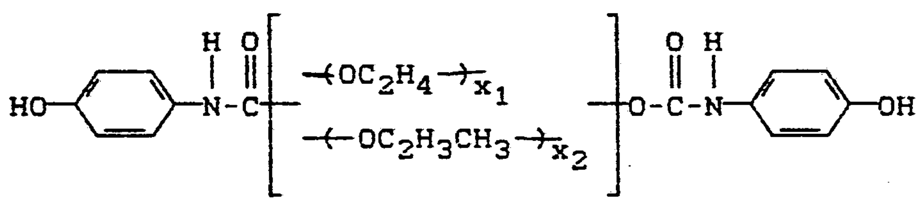

(21)

$x_1$ = 1 bis 200, $x_2$ = 1 bis 100, $x_1+x_2$ = 4 bis 200,

als statistisch verteiltes Copolymer oder als Block-Copolymer.

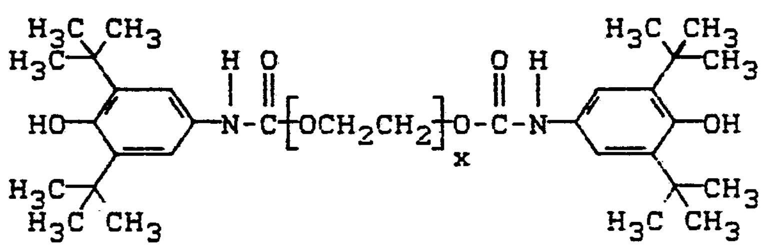

(22)

x = 3 bis 220, vorzugsweise 3 bis 10.

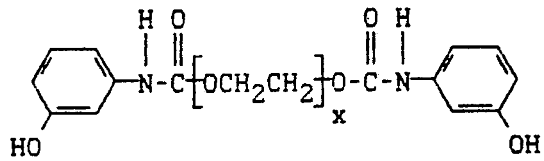

(23)

x = 3 bis 220, vorzugsweise 3 bis 10.

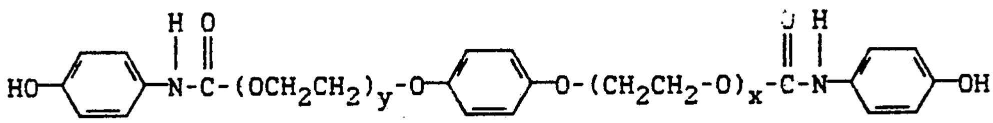

(24)

x und y gleich oder verschieden, x + y = 2 bis 200, vorzugsweise x = y = 1 oder 2.

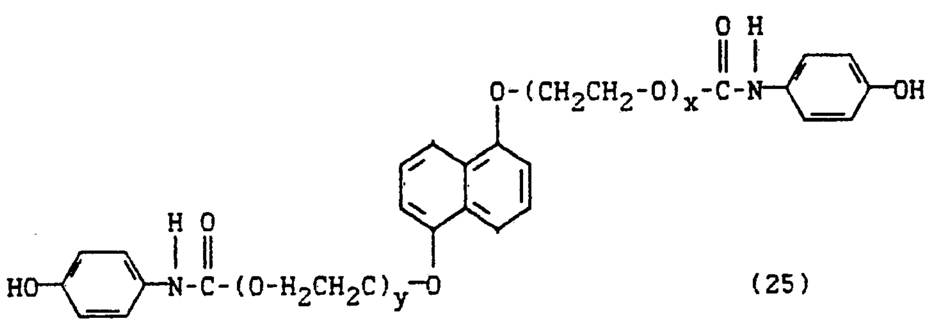

(25)

x und y gleich oder verschieden, x + y = 2 bis 200, vorzugsweise x = y = 1 oder 2.

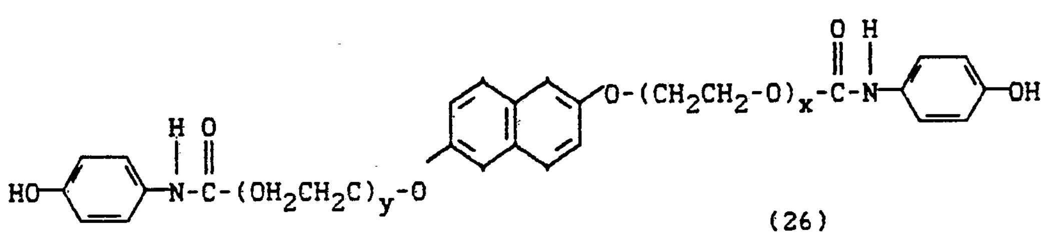

(26)

x und y gleich oder verschieden, x + y = 2 bis 200, vorzugsweise x = y = 1 oder 2.

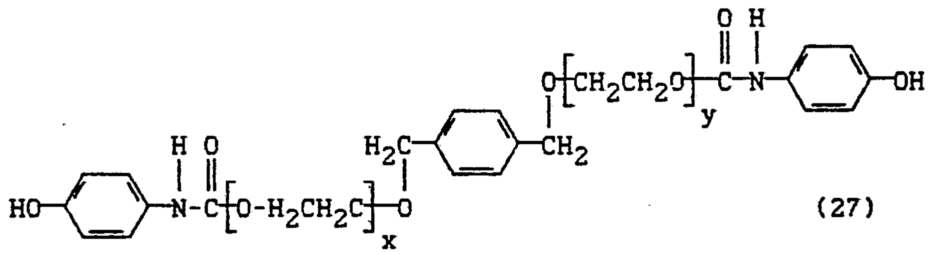

(27)

x und y gleich oder verschieden, x + y = 2 bis 200, vorzugsweise x = y = 1 oder 2.

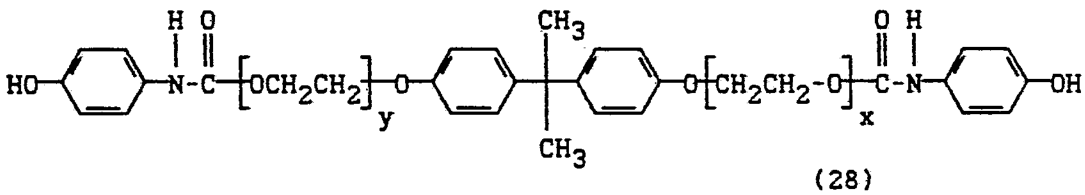

(28)

x und y gleich oder verschieden, x + y = 2 bis 200, vorzugsweise x = y = 1 oder 2.

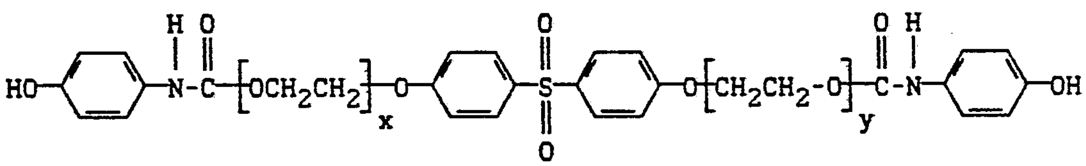

(29)

x und y gleich oder verschieden, x + y = 2 bis 200, vorzugsweise x = y = 1 oder 2.

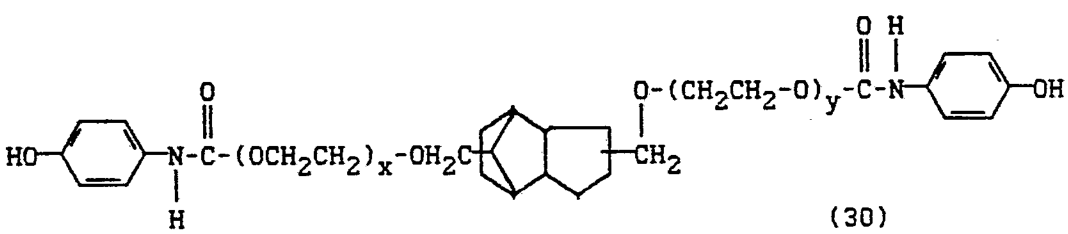

(30)

x und y gleich oder verschieden, x + y = 2 bis 200, vorzugsweise x = y = 1 oder 2.

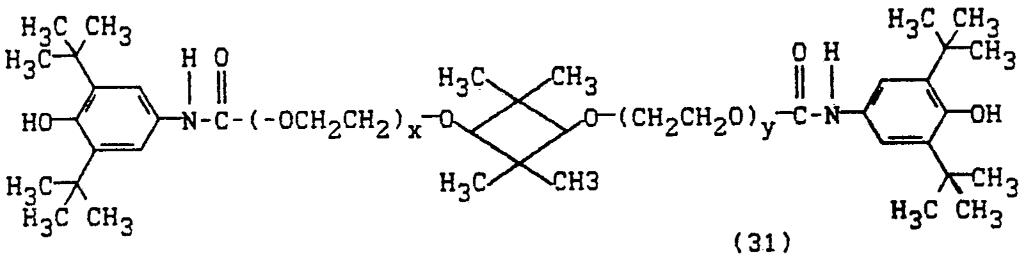

(31)

x und y gleich oder verschieden, x + y = 2 bis 200, vorzugsweise x = y = 1 oder 2, Konfiguration am Cyclobutanring: cis und/oder trans.

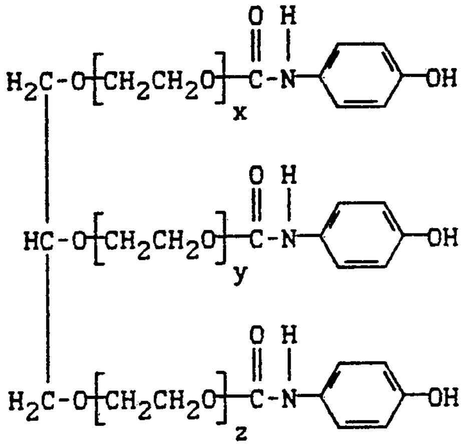

(32)

x, y und z gleich oder verschieden, x + y + z = 2 bis 200, vorzugsweise x + y + z = 3 bis 20.

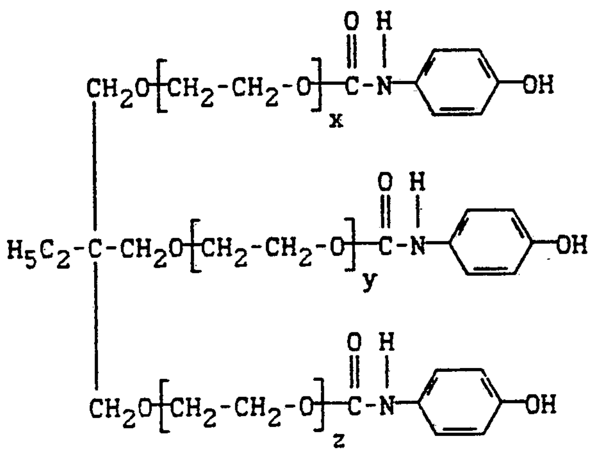

(33)

x, y und z gleich oder verschieden, x + y + z = 2 bis 200, vorzugsweise x + y + z = 3 bis 20.

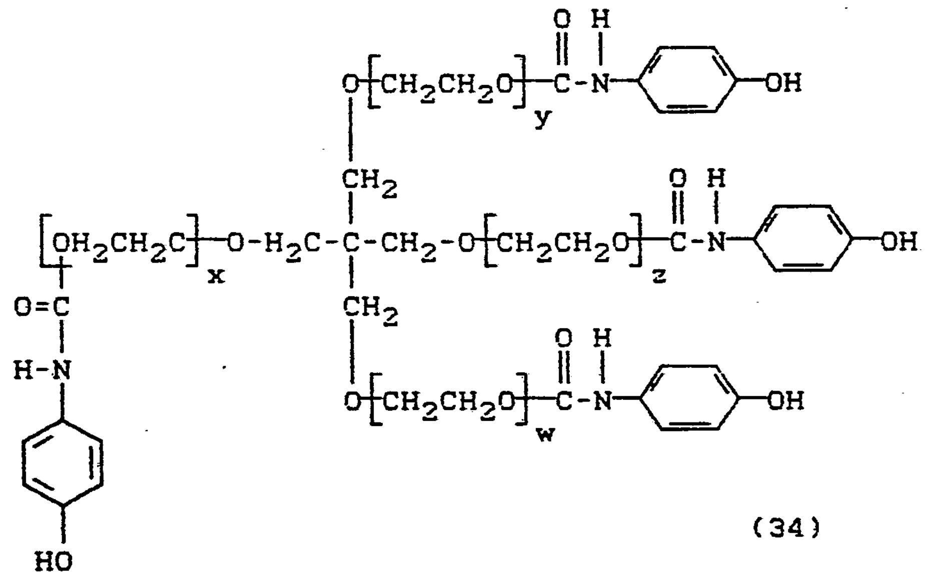

(34)

w, x, y und z gleich oder verschieden, w + x + y + z = 2 bis 200, vorzugsweise w + x + y + z = 4 bis 20.

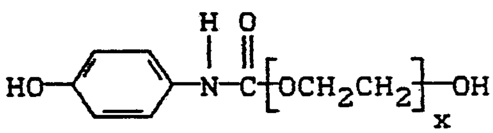

(35)

x = 3 bis 220, vorzugsweise x = 3 bis 10.

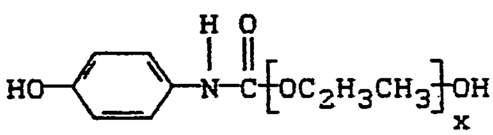

(36)

x = 3 bis 170, vorzugsweise x = 3 bis 10.

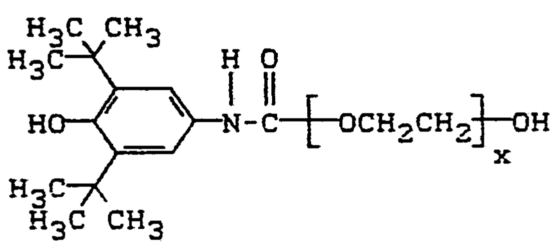

(37)

x = 3 bis 220, vorzugsweise x = 3 bis 10.

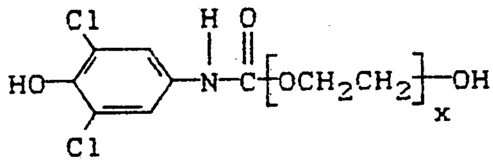

(38)

x = 3 bis 220, vorzugsweise x = 3 bis 10.

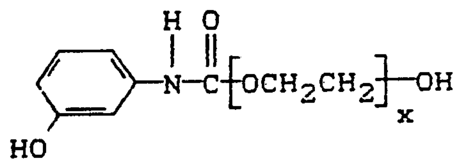

(39)

x = 3 bis 220, vorzugsweise x = 3 bis 10.

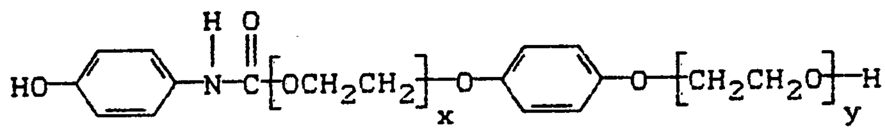

(40)

x und y gleich oder verschieden, x + y = 2 bis 200, vorzugsweise x = y = 1 oder 2.

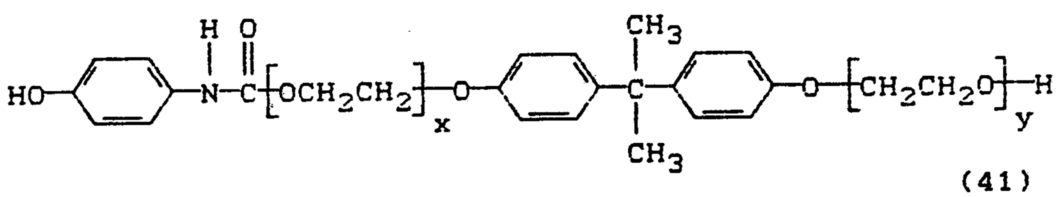

(41)

x und y gleich oder verschieden, x + y = 2 bis 200, vorzugsweise x = y = 1 oder 2.

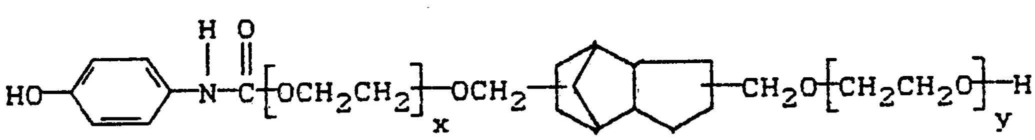

(42)

x und y gleich oder verschieden, x + y = 2 bis 200, vorzugsweise x = y = 1 oder 2.

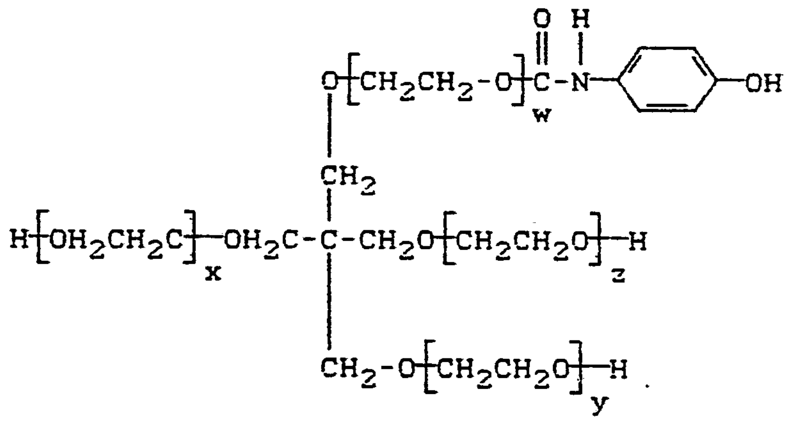

(43)

w, x, y und z gleich oder verschieden, w + x + y + z = 2 bis 200, vorzugsweise w + x + y + z = 4 bis 20.

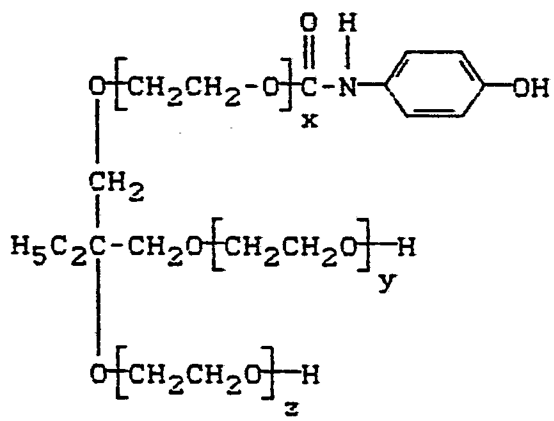

(44)

x, y und z gleich oder verschieden, x + y + z = 2 bis 200, vorzugsweise x + y + z = 3 bis 20.

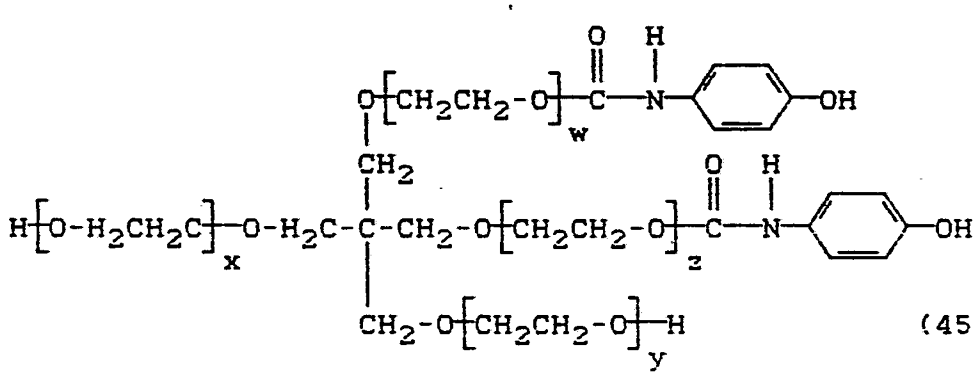

(45)

w, x, y und z gleich oder verschieden, w + x + y + z = 2 bis 200, vorzugsweise w + x + y + z = 4 bis 20.

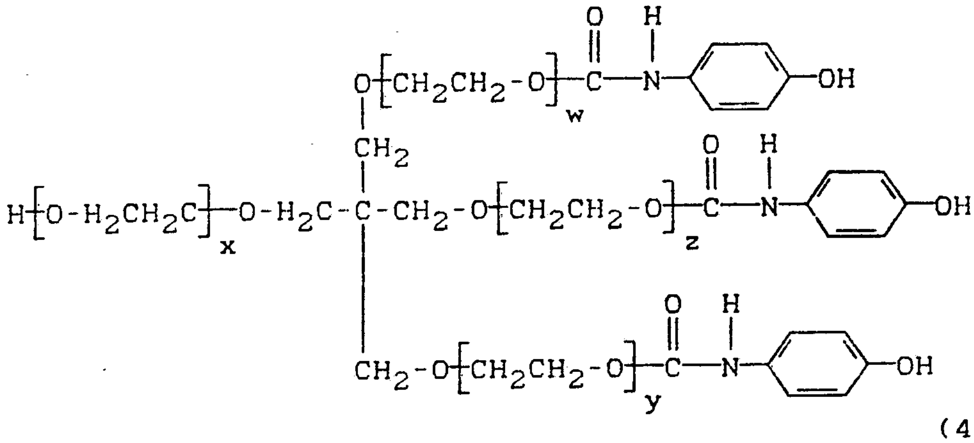

(46)

w, x, y und z gleich oder verschieden, w + x + y + z = 2 bis 200, vorzugsweise w + x + y + z = 4 bis 20.

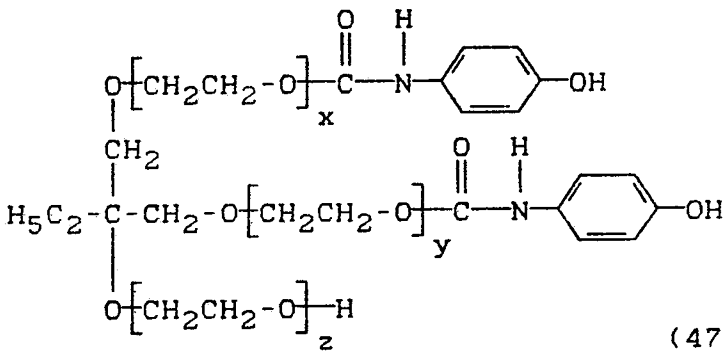

(47)

x, y und z gleich oder verschieden, x + y + z = 2 bis 200, vorzugsweise x + y + z = 3 bis 20.

Bevorzugt werden erfindungsgemäß auch solche Hydroxyphenylurethane der Formel (3) hergestellt, die den zuvor angeführten entsprechen, bei denen jedoch R' nicht für Wasserstoff, sondern für Alkyl oder Phenyl steht, insbesondere für Alkyl und ganz besonders für Methyl oder Ethyl.

Besonders bevorzugt werden erfindungsgemäß folgende Hydroxyphenylurethane der Formel (3) hergestellt: α,ω-Bis-(4-hydroxyphenylcarbamoyloxy)-n-alkan mit $C_8$-$C_{20}$-n-alkan, 1,3-Bis-(4-hydroxyphenylcarbamoyloxy)-2-methyl-2-(1-propyl)-propan, 1,3-Bis-(4-hydroxyphenylcarbamoyloxy)-2,2-diethylpropan, 1,3-Bis-(4-hydroxyphenyl-carbamoyloxy)-2-ethylhexan, 1,3-Bis-(4-hydroxyphenylcarbamoyloxy)-2,2,4-trimethylpentan, 1,12-Bis-(4-hydroxyphenylcarbamoyloxy)-octadecan, 1,12-Bis-(3,5-di-tert.-butyl-4-hydroxyphenylcarbamoyloxy)-octadecan, α,ω-Bis-(4-hydroxy-3,5-trimethylphenyl-carbamoyloxy)-n-alkan mit $C_8$-bis $C_{20}$-n-alkan, 1,12-Bis-(3-hydroxyphenylcarbamoyloxy)-octadecan, α,ω-Bis-(3-hydroxyphenylcarbamoyloxy)-n-alkan mit $C_8$- bis $C_{20}$-n-alkan, α-(4-Hydroxyphenylcarbamoyloxy)-ω-hydroxy-n-alkan mit $C_6$- bis $C_{20}$-n-alkan, 1-(4-Hydroxyphenylcarbamoyloxy)-2,2-diethyl-3-hydroxypropan, 1-(4-Hydroxyphenylcarbamoyloxy)-3-hydroxy-2-methyl-2-(1-propyl)-propan, 1-(4-Hydroxyphenylcarbamoyloxy)-2-ethyl-3-hydroxyhexan, 6-(4-Hydroxyphenylcarbamoyloxy)-1-hydroxy-2,2,4-trimethylhexan, 1-(4-Hydroxyphenylcarbamoyloxy)-12-hydroxyoctadecan, α-(3,5-Di-tert.-butyl-4-hydroxyphenylcarbamoyloxy)-ω-hydroxy-n-alkan mit $C_6$- bis $C_{20}$-n-alkan, 1-(3,5-Di-tert.-butyl-4-hydroxyphenylcarbamoyloxy)-12-hydroxyoctadecan, α-(3-Hydroxyphenylcarbamoyloxy)-ω-hydroxy-n-alkan mit $C_6$- bis $C_{20}$-n-Alkan, 1-(3-Hydroxyphenylcarbamoyloxy)-12-hydroxyoctadecan, sowie solche der Formeln (19) bis (22), (24) bis (27), (31) bis (33), (35) bis (37) und (44). Ganz besonders sind unter diesen Verbindungen und Verbindungstypen solche mit Molekulargewichten bis zu 1200 bevorzugt.

Das erfindungsgemäße Verfahren kann beispielsweise bei Temperaturen im Bereich von 80 bis 250°C durchgeführt werden. Optimale Temperaturbereiche sind im allgemeinen abhängig von der Art der Einsatzstoffe und gegebenenfalls einfach zu ermitteln. Bevorzugt wird das erfindungsgemäße Verfahren bei Normaldruck durchgeführt, es kann jedoch auch bei vermindertem oder erhöhtem Druck durchgeführt werden, beispielsweise bei Drucken zwischen 10 mbar und 200 bar, vorzugsweise bei Drucken im Bereich von 0,5 bis 10 bar.

Es ist möglich, das erfindungsgemäße Verfahren in Abwesenheit von Lösungsmitteln durchzuführen, beispielsweise mit geschmolzenen Einsatzmaterialien. Insbesondere ist dies möglich, wenn Produkte der Formel (3) mit m = 1 hergestellt werden sollen und der Alkohol der Formel (2) im großen Überschuß angewendet wird. Vorzugsweise wird das erfindungsgemäße Verfahren jedoch in Gegenwart von Lösungsmitteln durchgeführt, die zumindest den einzusetzenden Alkohol der Formel (2) bei Reaktionstemperatur zu lösen vermögen und deren Siedetemperatur im Bereich der gewünschten Reaktionstemperatur oder darüber liegt, beispielsweise im Bereich 100 bis 300°C.

Als Lösungsmittel kommen beispielsweise infrage Toluol, Xylole, Ethylbenzol, Isooctan, Isododecan, Kohlenwasserstofffraktionen im Siedebereich von 100 bis 300°C, Anisol, Diphenylether, Tetramethylensulfon, Acetonitril, Benzonitril, N,N-Dimethylacetamid, N-Methylpyrrolidon, N-Methyl-ε-caprolactam, Diethylenglykoldimethylether, Diethylenglykoldiethylether, Dichlorbenzole und Dimethylsul-

foxid. Beim Arbeiten unter Druck können auch bei Normaldruck niedriger siedende Lösungsmittel eingesetzt werden. Geeignete Lösungsmittelmengen sind beispielsweise solche im Bereich von 20 bis 95 Gew.-%, bezogen auf das gesamte Reaktionsgemisch. Vorzugsweise beträgt diese Menge 30 bis 70 Gew.-%.

Das erfindungsgemäße Verfahren kann gegebenenfalls in Gegenwart von Katalysatoren durchgeführt werden. Geeignete Katalysatoren sind beispielsweise Lewis-Säuren, Brönsted-Säuren, Lewis-Basen, Brönsted-Basen und Gemische (Salze) von Säuren und Basen. Bevorzugte Katalysatoren sind solche der nachfolgend angeführten Gruppen A und B. Die Katalysatoren können auch in beliebigen Gemischen aus einer oder beiden Gruppen verwendet werden.

Katalysatoren der Gruppe A umfassen tertiäre Amine und tertiäre Phosphine, beispielsweise Triethylamin, Tri-n-butylamin, Tricyclohexylamin, N,N-Diethylanilin, N,N-Diethylanilin, N,N,N',N'-Tetramethyl-1,2-ethylendiamin, Pyridin, Picoline, Lutidine, Chinolin, Isochinolin, 1,4-Diazabycyclo-[2,2,2]-octan, 2,2'-Dipyridyl, N,N-Dimethyl-4-aminopyridin, Chinazolin, Pyrazin, Triphenylphosphin, P,P,P',P'-Tetraphenyl-1,3-diphosphinopropan, Bis-(dimethylamino)-methylphosphin und Tris-(4-dimethylaminophenyl)-phosphin, sowie polymer gebundene tertiäre Amine oder Phosphine wie Styrol-Vinylpyridin-Copolymere oder polymer gebundene Triphenylphosphine wie sie in der US-PS 3 708 462 beschrieben sind. Bevorzugte Katalysatoren der Gruppe A sind Chinolin und Isochinolin. Die Katalysatoren

der Gruppe A können beispielsweise in Mengen von 0,5 bis 10 Gew.-%, vorzugsweise von 0,5 bis 5 Gew.-%, bezogen auf das gesamte Reaktionsgemisch (gegebenenfalls einschließlich Lösungsmittel) verwendet werden.

Die Katalysatoren der Gruppe B umfassen metallorganische Verbindungen, insbesondere Alkoxyverbindungen, z.B. Metallsäureester, und Carboxylate der Elemente Lithium, Natrium, Kalium, Magnesium, Calcium, Aluminium, Thallium, Titan und Zinn, wobei die organischen Reste pro Metallvalenz beispielsweise 1 bis 20 C-Atome enthalten können. Ferner sind Oxide dieser Metalle geeignet. Bevorzugte Katalysatoren der Gruppe B sind solche mit einer Zusammensetzung wie in der EP-OS 48 368 oder der US-PS 4 018 744 beschrieben. Beispiele für Katalysatoren der Gruppe B sind Lithiumacetat, Lithiumnaphtenat, Kaliumacetat, Kaliumstearat, Natriumacetat, Natriumpropionat, Magnesiumoleat, Calciumcitrat, Aluminiumtriisopropylat, Diethylaluminiumethylat, Thallium-(I)-acetat, Titan-tetra-n-butylat, Titan-(IV)-diisopropylat-bis-(2,4-pentandionat), Titan-(IV)-cresylat, Di-n-butyl-zinn-maleat, Di-n-butyl-zinndiacetat, Di-n-butyl-zinndilaurat und Di-n-butyl-zinndimethoxid. Die Katalysatoren der Gruppe B können beispielsweise in Mengen von 0,01 bis 2 Gew.-%, vorzugsweise 0,05 bis 0,8 Gew.-%, bezogen auf das gesamte Reaktionsgemisch (gegebenenfalls einschließlich Lösungsmittel) eingesetzt werden.

Zum Einsatz in das erfindungsgemäße Verfahren geeignete Polyurethane, die unter die Formel (4) fallen, können beispielsweise gemäß der EP-OS 94 861 durch Carbonylierung von Nitrophenolen oder nach dort zitierten Literaturstellen erhalten werden. Ferner können sie aus Hydroxyphenylurethanen mit kleinen O-Alkylresten durch Abdestillieren des Alkohols, der diesen kleinen Alkylrest enthält, hergestellt werden (siehe Beispiel 11.1) oder sie können in ähnlicher Weise in einer in-situ-Variante erzeugt und unmittelbar weiter umgesetzt werden (siehe Beispiel 11.4). Andere Polyurethane, die unter die Formel (4) fallen, können beispielsweise durch HCl-Abspaltung aus den entsprechenden N-substituierten Hydroxyphenylcarbamoylchloriden oder direkt durch Phosgenierung von N-substituierten Aminophenolen erhalten werden.

Für die erfindungsgemäße Umsetzung von Polyurethanen der Formel (4), mit Alkoholen der Formel (2) kann das Verhältnis der Reaktanten beispielsweise so gewählt werden, daß auf ein Mol des Alkohols der Formel (2) m Mole der repetierenden Einheit der Formel (1) im Polyurethan der Formel (4) zur Verfügung stehen. Von diesem Verhältnis kann beispielsweise auch um ± 20 % abgewichen werden. In denjenigen Fällen, in denen Hydroxyphenylurethane der Formel (3) mit m = 1 hergestellt werden kann der Alkohol der Formel (2) auch in weit größerem Überschuß eingesetzt werden, beispielsweise bis zum 100fachen der stöchiometrischen Menge. Der Alkohol der Formel (2) kann dann nicht nur als Reaktant sondern auch als Lösungsmittel dienen.

Umsetzungen von Polyurethanen der Formel (4) mit Alkoholen der Formel (2) werden vorzugsweise im Temperaturbereich von 100 bis 200° C durchgeführt. Man kann dabei so vorgehen, daß man die Reaktionsteilnehmer, gegebenenfalls eines oder mehrere Lösungsmittel und gegebenenfalls einen oder mehrere Katalysatoren von Anfang an vorlegt und in einem Zeitraum von beispielsweise 0,5 bis 24 Stunden umsetzt. Es ist jedoch auch möglich einen der Reaktionspartner, gegebenenfalls den Katalysator und/oder gegebenenfalls das Lösungsmittel in Portionen nach und nach zuzugeben.

Nach dem Ende der Umsetzung, das beispielsweise durch Verfolgung der Konzentration des Alkohols der Formel (2) festgestellt werden kann, kann das Reaktionsgemisch auf verschiedene Weise aufgearbeitet werden. Häufig liegen die Reaktionsprodukte suspendiert vor oder scheiden sich aus dem Reaktionsgemisch beim Abkühlen oder Einengen als Festprodukt ab. Sie können dann beispielsweise durch Filtration isoliert werden. Wenn ein Lösungsmittel verwendet worden ist, ist es häufig vorteilhaft dieses vor der eigentlichen Aufarbeitung zu entfernen, beispielsweise durch Destillation.

Die so erhaltenen festen, flüssigen oder viskosen Rohprodukte der Formel (3) können gegebenenfalls weiter gereinigt werden. Beispielsweise können durch Waschen mit Salzsäure, Wasser und/oder Methanol Katalysatorreste entfernt werden. Beispielsweise können durch selektives Auswaschen, Extraktion, Umkristallisation, Sublimation und/oder Umfällen gegebenenfalls vorhandene Verunreinigungen sowie eventuell vorhandene unumgesetzte Ausgangsprodukte abgetrennt werden. Falls gewünscht, kann mit derartigen Methoden auch häufig erreicht werden, daß zunächst isolierte Gemische von verschiedenen Individuen der Formel (3) in die Einzelverbindungen aufgetrennt werden.

Bei einer anderen Variante des erfindungsgemäßen Verfahrens werden Hydroxyphenylurethane der Formel (5) mit Alkoholen der Formel (2) umgesetzt. Bei dieser Variante setzt man vorzugsweise m Mole Hydroxyphenylurethan der Formel (5) auf ein Mol Alkohol der Formel (2) ein. Abweichungen von diesem Verhältnis von beispielsweise ± 20 % sind jedoch auch möglich. Auch bei dieser Variante kann der Alkohol der Formel (2) in großem Überschuß eingesetzt und gleichzeitig auch als Lösungsmittel verwendet werden, wenn man Hydroxyphenylurethane der Formel (3) herstellen will, bei denen m = 1 ist. Als Einsatzstoffe des erfindungsgemäßen Verfahrens sind solche Hydroxyphenylurethane der Formel (5) besonders geeignet, bei denen $R_5$ für Ethyl, 1-Propyl oder 2-Propyl steht, da dann der bei der Umsetzung entstehende Alkohol ($R_5$-OH) während der Reaktion leicht abdestilliert werden kann. Die Reaktionstemperaturen und -zeiten, sowie die Aufarbeitung des Reaktionsgemisches kann so erfolgen wie oben bei der Umsetzung von Polyurethanen der Formel (4) mit Alkoholen der Formel (2) beschrieben.

In einer weiteren Variante des erfindungsgemäßen Verfahrens werden Hydroxyphenylurethane der Formel (6) mit Alkoholen der Formel (2) umgesetzt zu Hydroxyphenylurethanen der Formel (3), bei denen n von Null verschieden ist und vorzugsweise für 1 steht und besonders bevorzugt n und m für 1 stehen. Bei dieser Variante reagieren $\frac{m}{m+n}$ Mole Verbindung der Formel (6) mit $\frac{n}{m+n}$ Molen Verbindung der

Formel (2). Die Einsatzstoffe der Formeln (6) und (2) werden vorzugsweise in diesem Verhältnis zueinander eingesetzt oder in Verhältnissen, die um bis zu ± 20 % davon abweichen. Wenn Produkte der Formel (3) hergestellt werden sollen, in denen m für 1 steht, kann auch hier der Alkohol der Formel (2) in weit größerem Überschuß verwendet werden und gleichzeitig als Lösungsmittel dienen.

Die für diese Verfahrensvariante benötigten Einsatzstoffe der Formel (6) können selbst Produkte des erfindungsgemäßen Verfahrens sein. Deshalb ist es vorteilhaft diese Verfahrensvariante als Eintopfreaktion durchzuführen, indem in einer ersten Stufe, vorzugsweise aus Verbindungen der Formeln (5) und (2) ein Produkt der Formel (6) erzeugt und dieses ohne Isolierung weiter umgesetzt wird (siehe auch Beispiel 8).

Die sonstigen Reaktionsbedingungen können beispielsweise so gewählt werden, wie zuvor für die Umsetzung von Verbindungen der Formeln (4) und (2) beschrieben. Ebenso kann die Aufarbeitung wie dort beschrieben durchgeführt werden.

Schließlich gibt es noch eine Variante des erfindungsgemäßen Verfahrens, bei der symmetrisch substituierte Harnstoffe der Formel (7) mit Alkoholen der Formel (2) umgesetzt werden. Die Ausgangsprodukte der Formel (7) können beispielsweise entsprechend der von C.Giori, Polym. Prep. 11, 328 (1970) beschriebenen Methode hergestellt werden oder durch Umsetzung von Hydroxyphenylisocyanten oder Hydroxyphenylcarbaminsäurechloriden mit den entsprechenden Aminophenolen.

Die Umsetzung von Harnstoffen der Formel (7) mit Alkoholen der Formel (2) erfolgt so, daß m Mole Harnstoff der Formel (7) mit einem Mol Alkohol der Formel (2) zu einem Mol Hydroxyphenylurethan der Formel (3) reagieren, wobei als Nebenprodukt m Mole eines Aminophenols entstehen. Die Einsatzstoffe der Formeln (7) und (2) können in diesem stöchiometrischen Verhältnis eingesetzt werden, oder in Verhältnissen, die um beispielsweise bis zu ± 20 % davon abweichen. Auch hier kann der Alkohol der Formel (2) im großen Überschuß eingesetzt werden und gleichzeitig als Lösungsmittel dienen, wenn Verbindungen der Formel (3) mit m = 1 hergestellt werden sollen.

Diese Verfahrensvariante wird vorzugsweise bei Temperaturen im Bereich 150 bis 220°C durchgeführt. Die sonstige Reaktionsführung und die Aufarbeitung kann so erfolgen, wie zuvor bei der Umsetzung von Verbindungen der Formel (4) mit Alkoholen der Formel (2) beschrieben. Das als Koppelprodukt anfallende Aminophenol wird vorzugsweise mit verdünnter Salzsäure als Hydrochlorid abgetrennt, zurückgewonnen, und für die Herstellung des Harnstoffs der Formel (7) wieder verwendet. Bei der erfindungsgemäßen Umsetzung von Verbindungen der Formel (7) mit Verbindungen der Formel (2) können gegebenenfalls Katalysatoren zugesetzt werden. Geeignete Katalysatoren sind beispielsweise in den DE-OS'en 2 943 550, 2 943 551 und 2 258 454 beschrieben.

Von den oben beschriebenen Varianten des erfindungsgemäßen Verfahrens sind diejenigen bevorzugt, bei denen Verbindungen der Formeln (5) oder (6) mit Alkoholen der Formel (2)

umgesetzt werden. Als Einsatzstoffe der Formel (5) werden bevorzugt solche verwendet, die nach dem Verfahren der DE-OS 3 406 230 hergestellt wurden.

Es ist ausgesprochen überraschend, daß es erfindungsgemäß in einem Verfahren mit unproblematischer Handhabung der Einsatzstoffe gelingt, Hydroxyphenylurethane der Formel (3) in guten Ausbeuten herzustellen, da in den Verfahrensprodukten selbst Umesterungsreaktionen zugängliche phenolische oder alkoholische Hydroxylgruppen vorliegen. Es war somit mit umfangreicher Nebenproduktbildung und erschwerter Trennung der Reaktionsprodukte zu rechnen.

Die Produkte des erfindungsgemäßen Verfahrens können als Stabilisatoren und als Monomere für die Herstellung von Polykondensaten und Polyaddukten verwendet werden.

Die nachstehenden Beispiele zeigen mögliche Ausführungsformen des erfindungsgemäßen Verfahrens ohne die Erfindung auf diese Ausführungsformen zu beschränken.

Beispiel 1

Herstellung von Bisurethanen durch Umesterung mit α,ω-Alkandiolen in Gegenwart von Chinolin als Katalysator

In einer Rührapparatur mit 20 cm Vigreux-Kolonne und Destillationsaufsatz wurden 21,8 g (0,125 Mol) 1,10-Decandiol und 45,3 g (0,250 Mol) N-(4-Hydroxyphenyl)-ethylcarbamat in 300 g ortho-Dichlorbenzol vorgelegt und 2 Stunden am Rückfluß erhitzt. Danach wurde anfallendes Ethanol abdestilliert. Bei nachlassendem Ethanolanfall - ca. 4 Stunden nach Beginn der Abnahme - wurden 3,8 g Chinolin zugegeben, wonach wiederum Ethanol abdestilliert wurde. Nachdem kein Ethanol mehr überging, wurde abgekühlt, der ausgefallene Feststoff abgesaugt und dieser in 200 ml Aceton aufgerührt. Nach Absaugen und Trocknen wurden 47,8 g (= 86 % Ausbeute) 1,10-Bis-(4-hydroxyphenylcarbamoyloxy)-decan mit einem Schmelzpunkt von 198° C erhalten.

In gleicher Weise wurden weitere α,ω-Alkandiole zu Bisurethanen umgesetzt (Ergebnisse siehe Tabelle 1).

Tabelle 1

Reaktionsprodukt:

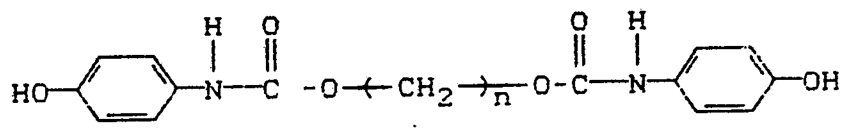

| Beispiel Nr. | n | Ausbeute % | Schmelzpunkt° C |
|---|---|---|---|
| 1.1 | 4 | 88 | 219-220 |
| 1.2 | 6 | 86 | 220-225 |
| 1.3 | 12 | 88 | 187 |
| 1.4 | 16 | 83 | 188 |
| 1.5*) | 19 | 83 | 173-176 |

*) Zur Herstellung des eingesetzten $C_{19}$-Diols siehe Beispiel 3.

Beispiel 2

Nach der Arbeitsweise von Beispiel 1 wurden äquimolare Mengen von weiteren Diolen zu den entsprechenden Bisurethanen umgesetzt, Ergebnisse siehe Tabelle 2.

Die in den Beispielen 2.4 bis 2.7 eingesetzten Diolkomponenten wurden durch Ethoxylierung der entsprechenden phenolischen Dihydroxyverbindungen erhalten.

Tabelle 2

| Beispiel Nr. | Reaktionsprodukt 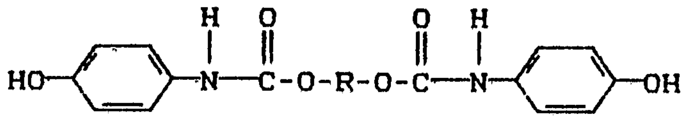 | Ausbeute % | Schmelzpunkt °C |
|---|---|---|---|
| 2.1 | R = 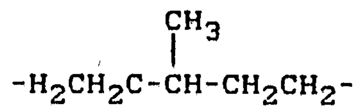 | 78 | 158 |
| 2.2 | R = 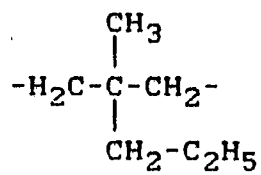 | 55 | 96-100 |
| 2.3 | R = 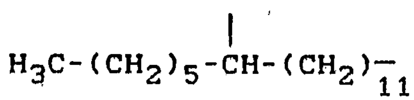 | 88 | 101-105 |
| 2.4 | R = 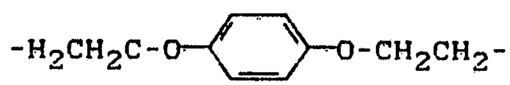 | 80 | 253 |
| 2.5 | R = 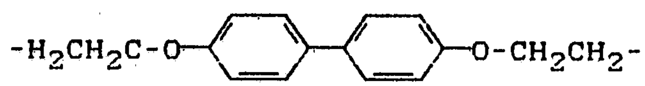 | 83 | 233-239 |

Tabelle 2 (Fortsetzung)

| Beispiel Nr. | Reaktionsprodukt 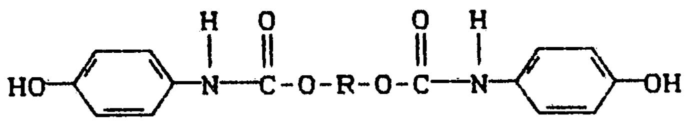 | Ausbeute % | Schmelzpunkt °C |
|---|---|---|---|
| 2.6 | R = 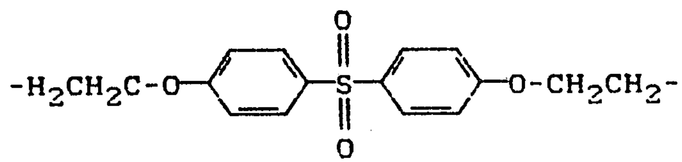 | 77 | 222-225 |
| 2.7 | R = 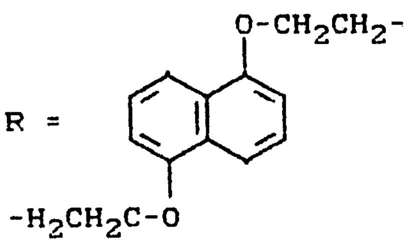 | 83 | 278-82 |

Beispiel 3

Nach der Arbeitsweise von Beispiel 1 wurden 22,5 g (0,075 Mol) "Diol verzweigt" (Herstellung siehe unten) mit 27,15 g (0,15 Mol) N-(4-Hydroxyphenyl)-ethylcarbamat umgesetzt. Die erhaltene Reaktionslösung wurde von einem zähviskosen Produkt abgegossen und dieses mit 200 ml Methylenchlorid aufgerührt. Hierbei wurden 4,5 g eines in Methylenchlorid unlöslichen Feststoffes vom Schmelzpunkt 115 bis 132°C erhalten (Produkt A).

Die Methylenchloridphase und die ortho-Dichlorbenzolphase wurden vereinigt, mit 4 %iger Salzsäure gewaschen und durch Stehen über $Na_2CO_3$ entsäuert und getrocknet. Nach Entfernen der Lösungsmittel wurden 26,7 g eines rötlichen, zunächst zäh-viskosen, später kristallisierenden Produkts vom Schmelzpunkt 50 bis 55°C erhalten (Produkt B).

Die beiden Produkte A und B sind nach ihrer analytischen Charakterisierung Isomere bzw. Isomerengemische verschiedener Bis-(4-hydroxyphenylcarbamoyloxy)-nonadecane. Ausbeute (Summe der Produkte A und B): 73 %.

Herstellung der $C_{19}$-Diole

a) $C_{19}$-Dicarbonsäuredimethylester (Heptadecandicarbonsäuredimethylester) wurden durch Hydrocarboxymethylierung von Ölsäuremethylester unter folgenden

Bedingungen gewonnen (siehe auch B. Gruber und M. Biermann; Fette, Seifen, Anstrichmittel **87** (1985) 10, S. 400-403):

Einsatzstoffe (Mole):

| | |
|---|---|
| Ölsäuremethylester | (25) |
| Methanol | (50) |
| Pyridin | (15) |
| $Co_2(CO)_8$ | (0,5) |
| Kohlenmonoxid | (1,5), 2 Vol-% $H_2$ enthaltend. |

Reaktionsbedingungen:
150 bar CO (Anfangsdruck bei Raumtemperatur)
185° C Reaktionstemperatur
10 Stunden Reaktionszeit

Zur Zerstörung des Katalysators wurde das Reaktionsgemisch in Gegenwart von verdünnter Essigsäure bei 50° C mit Luft behandelt und dann mit dem gleichen Volumen Wasser versetzt. Die organische Phase wurde von Methanol befreit und an einer 1 m Füllkörperkolonne fraktioniert. Die bei 190 bis 200° C (1 mbar) übergehende Fraktion wurde weiterverwendet ("Diester-Isomerengemisch").

Zur Trennung wurde das Diester-Isomerengemisch zweimal aus Methanol umkristallisiert. Hierbei wurden pro 100 g Substanz jeweils 2 l Methanol verwendet. Das Kristallisat war ein Feststoff mit einem Schmelzpunkt von 60-61° C, welches zu 90,5 % aus dem linearen $\alpha,\omega$-Diester bestand ("Diester unverzweigt").

Aus der methanolischen Mutterlauge wurde durch Abdestillieren des Methanols ein bei Raumtemperatur flüssiges Produkt erhalten, welches zu 96,5 % aus verzweigten $C_{19}$-Diestern ("Diester verzweigt") besteht.

b) $C_{19}$-Diole wurden durch Hydrierung der $C_{19}$-Diester nach Adkins hergestellt. Hierzu wurden die Diester in Gegenwart von 15 Gew.-% pulverförmigem Kupfer-Bariumchromit-Katalysator (48 Gew.-% CuO, 10 Gew.-% BaO und 37 Gew.-% $Cr_2O_3$) bei 250°C unter 250 bar $H_2$ (Anfangsdruck bei Raumtemperatur) innerhalb von 18 Stunden hydriert.

Aus "Diester unverzweigt" wurde so nach Umkristallisation aus Toluol in Gegenwart von Aktivkohle 1,19 - Nonadecandiol mit einem Schmelzpunkt von 95 bis 96°C in 80 % Ausbeute (bezogen auf eingesetzten Diester) erhalten. Dieses wurde in Beispiel 1.5 eingesetzt. Der "Diester verzweigt" wurde in der gleichen Weise hydriert, in Toluol aufgenommen und heiß über Aktivkohle filtriert. Nach dem Entfernen der Lösungsmittel wurde am Rotationsverdampfer bei 0,8 mbar das Diol mit einem Siedepunkt von 196°C erhalten, welches bei Raumtemperatur teils fest, teils flüssig vorliegt. Die Ausbeute an "Diol verzweigt" betrug 85 %, bezogen auf eingesetzten Diester. "Diol verzweigt" wurde in Beispiel 3 eingesetzt.

## Beispiel 4

Herstellung von Bisurethanen durch Umesterung mit Polyglykolen als Diolkomponente:

Nach der Arbeitsweise von Beispiel 1 wurden 18,8 g (0,125 Mol) 1,8-Dihydroxy-3,6-dioxaoctan (Triethylenglykol) mit 45,3 g (0,250 Mol) N-(4-Hydroxyphenyl)-ethylcarbamat umgesetzt. Bei der Aufarbeitung wurde anstelle von Aceton mit warmem Methylenchlorid digeriert. Es wurden 44,7 g (85 % Ausbeute) 1,8-Bis-(4-hydroxyphenylcarbamoyloxy)-3,6-dioxaoctan in Form eines zäh-viskosen Produktes erhalten.

In der gleichen Weise wurden weitere Polyglykole zu Bisurethanen umgesetzt, Ergebnis siehe Tabelle 3.

Tabelle 3

Reaktionsprodukt

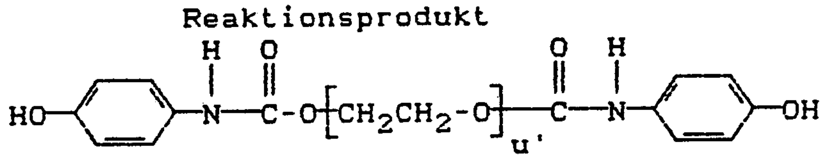

| Beispiel Nr. | u' | Ausbeute (%) | Schmelzpunkt (°C) oder Konsistenz |
|---|---|---|---|
| 4.1 | 2 | 83 | 129 |
| 4.2 | 4 | 85 | zäh-viskos |
| 4.3 | 9,5 | 81 | zäh-viskos |
| 4.4*) | 220 | 86 | 62-63 |

*) Ein von BP erhältliches Polyglykol (Breox® 8000) wurde, aus Aceton umkristallisiert, wie folgt charakterisiert ($M_n$: 9700, $M_w$ 10400, Uneinheitlichkeit: 0,072) und als Diol-Komponente eingesetzt.

Das Reaktionsprodukt wurde durch die 1 H-NMR-Spektroskopie als das gewünschte Bisurethan bestätigt. Die Hydroxyphenylcarbamoyl-Endgruppen wurden gegen tert.-Butanol als innerem Standard in einem Doppelresonanzexperiment (Unterdrückung des Singulettsignals der Methylenprotonen) quantitativ nachgewiesen.

Beispiel 5

Nach der Arbeitsweise von Beispiel 1 wurden 25,3 g (0,125 Mol) 1,12-Dodecandiol mit 45,3 g (0,25 Mol) N-(3-Hydroxyphenyl)-ethylcarbamat umgesetzt. Es wurden 49,0 g (83 % Ausbeute) 1,12-Bis-(3-hydroxyphenylcarbamoyloxy)-dodecan mit einem Schmelzpunkt von 144° C erhalten.

Beispiel 6

Nach der Arbeitsweise von Beispiel 1 wurden 25,3 g (0,125 Mol) 1,12-Dodecandiol mit 76,8 g (0,25 Mol) N-(3,5-di-tert.-butyl-4-hydroxyphenyl)-1-propylcarbamat unter Abdestillieren von 1-Propanol umgesetzt. Es wurden 61,1 g (67 % Ausbeute) 1,12-Bis-(3,5-di-tert.-butyl-4-hydroxyphenylcarbamoyloxy)-dodecan mit einem Schmelzpunkt von 87 bis 89° C erhalten.

Beispiel 7

(Umesterung mit Metallverbindungen als Katalysatoren)

Beispiel 7.1:

In einer Rührapparatur mit 20 cm Vigreux-Kolonne und Destillationsaufsatz wurden 13,8 g (0,08 Mol) 1,10-Decandiol, 28,7 g (0,16 Mol) N-(4-hydroxyphenyl)-ethylcarbamat und 0,3 g Dibutylzinndilaurat in 190 g ortho-Dichlorbenzol vorgelegt und 2 Stunden am Rückfluß erhitzt. Danach wurde erschöpfend Ethanol abdestilliert, was ca. 6 Stunden dauerte. Aus dem erkalteten Reaktionsgemisch wurde der ausgefallene Feststoff abgesaugt, mit 4 %iger Salzsäure und Wasser gewaschen. Danach wurde der Feststoff in 500 ml Aceton aufgerührt, abgesaugt und getrocknet.

Es wurden 26,7 g (= 76 % Ausbeute) 1,10-Bis-(4-hydroxyphenylcarbamoyloxy)-decan mit einem Schmelzpunkt von 196 bis 200°C erhalten.

Beispiel 7.2:

Es wurde wie in Beispiel 7.1 gearbeitet, jedoch wurden an Stelle von Dibutylzinndilaurat 0,3 g Di-butylzinnmethoxid verwendet. Das gewünschte Bisurethan wurde mit ähnlichem Ergebnis erhalten.

Beispiel 7.3:

Es wurde wie in Beispiel 7.1 gearbeitet, jedoch wurden an Stelle von Dibutylzinndilaurat 0,3 g Orthotitansäuretetrabutylester verwendet. Das gewünschte Bisurethan wurde mit ähnlichem Ergebnis erhalten.

Beispiel 8

Herstellung von Dihydroxymonourethanen nach einem speziellen Umesterungsverfahren mit α,ω-Alkandiolen in Gegenwart von Chinolin als Katalysator:

In einer Rührapparatur mit 20 cm Vigreux-Kolonne und Destillationsaufsatz wurden 21,8 g (0,125 Mol) 1,10-Decandiol und 45,3 g (0,250 Mol) N-(4-Hydroxyphenyl)-ethylcarbamat zusammen mit 3,8 g Chinolin in 300 g ortho-Dichlorbenzol vorgelegt und 25 Stunden am Rückfluß erhitzt. Danach wurde erschöpfend Ethanol abdestilliert (ca. 11 Stunden).

Zu der Reaktionslösung wurden weitere 45 g (0,258 Mol) 1,10-Decandiol hinzugefügt und 10 Stunden am Rückfluß erhitzt. Beim Erkalten kristallisierten 107,3 g Rohprodukt aus, welches aus Toluol umkristallisiert wurde. Das Kristallisat wurde zur Entfernung von 1,10-Decandiol mit heißem Methylenchlorid gewaschen. Nach dem Trocknen wurden 50,6 g (= 65,5 % Ausbeute bezogen auf eingesetztes Carbamat) 1-(4-Hydroxyphenylcarbamoyloxy)-10-hydroxydecan mit einem Schmelzpunkt von 110°C erhalten. Beim Umkristallisieren aus Toluol verblieben 12 g eines Toluolunlöslichen Rückstands, welcher im wesentlichen (nach 1 H-NMR) aus 1,10-Bis-(4-hydroxyphenylcarbamoyloxy)-decan bestand.

In der gleichen Weise wurden weitere α,ω-Alkandiole zu den gewünschten Dihydroxymonourethanen umgesetzt, Ergebnisse siehe Tabelle 4.

Tabelle 4

Reaktionsprodukt:

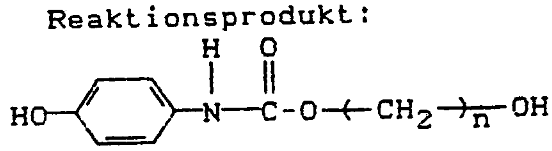

| Beispiel Nr. | n | Ausbeute % | Schmelzpunkt °C |
|---|---|---|---|
| 8.1*) | 6 | 81 | 126-30 |
| 8.2 | 12 | 69 | 155 |
| 8.3 | 16 | 74 | 151 |
| 8.4 | 19 | 72 | 148 |
| 8.5 | 4 | 75 | 129-30 |

*) Das Reaktionsgemisch wurde zuerst in Methylenchlorid, dann in 4 %iger Salzsäure aufgerührt, mit Wasser nachgewaschen und der erhaltene Feststoff getrocknet und aus n-Hexan umkristallisiert.

Beispiel 9

Nach der Arbeitsweise von Beispiel 8 wurden 45,3 g (0,250 Mol) N-(4-Hydroxyphenyl)-ethylcarbamat mit 35,8 g + 73,9 g (zusammen 0,383 Mol) 1,12-Octadecandiol umgesetzt und aufgearbeitet. Es wurden 74,8 g (= 71 % Ausbeute bezogen auf eingesetztes Carbamat) des gewünschten Reaktionsprodukts 1-(4-Hydroxyphenylcarbamoyloxy)-12-hydroxyoctadecan mit einem Schmelzpunkt von 123-25°C erhalten.

Als in Toluol unlöslicher Rückstand wurden 13,5 g des Dihydroxybisurethans (siehe Beispiel 2.3) erhalten.

Beispiel 10

Nach der Arbeitsweise von Beispiel 8 wurden 45,3 g (0,250 Mol) N-(4-Hydroxyphenyl)-ethylcarbamat mit 18,8 g + 38,7 g (zusammen 0,383 Mol) Triethylenglykol umgesetzt. Aus dem erkalteten Reaktionsgemisch wurde ein zäh-viskoses Produkt abgetrennt und dieses mehrfach in 50° C warmen Wasser aufgerührt und das Wasser abgegossen. Der Rückstand wurde in 1 l heißem Ethanol aufgenommen. Beim Erkalten fiel ein öliges Produkt aus, welches abgetrennt und getrocknet wurde. Es erwies sich als das gewünschte 1-(4-Hydroxyphenylcarbamoyloxy)-8-hydroxy-3,6-dioxaoctan. Ausbeute: 57,7 g = 81 %, bezogen auf eingesetzten Carbamat.

Beispiel 11

Dieses Beispiel beschreibt die Herstellung erfindungsgemäßer Urethane aus einem Polyurethan (polymeres Hydroxyphenylisocyanat).

Stufe 1); Beispiel 11.1:

Herstellung von Poly-[-4-(carbamoyloxy)-benzol].

In einer Rührapparatur mit 20 cm Vigreux-Kolonne und Destillationsaufsatz wurden in 570 g ortho-Dichlorbenzol 81,45 g (0,45 Mol) N-(4-Hydroxyphenyl)-ethylcarbamat am

Rückfluß erhitzt. Nach 5 Stunden wurde begonnen, Ethanol abzudestillieren. Als der Ethanolanfall nach 3 Stunden nachließ, wurden 30 g Chinolin hinzugefügt und es wurde erneut Ethanol abgenommen. Als nach insgesamt 20 Stunden Reaktionszeit die Reaktion beendet war, wurde abgekühlt, der ausgefallene Feststoff abgesaugt, gründlich mit Aceton gewaschen und getrocknet. Das Produkt war in Dimethylsulfoxid löslich. Die Charakterisierung durch die 1 H-NMR-Spektroskopie war in Übereinstimmung mit den Daten für Poly-[-4-(carbamoyloxy)-benzol] von EP 0 094 861, Beispiel 1. Ausbeute: 58 g = 95,4 % der Theorie.

Stufe 2a); Beispiel 11.2:

Herstellung von 1,10-Bis-(4-hydroxyphenyl-carbamoyloxy)-decan.

In einer Rührapparatur wurden 15 g (0,11 Mol als Monomer gerechnet) des nach Stufe 1) erhaltenen Polymers in einer Lösung von 8,7 g (0,05 Mol) 1,10-Decandiol, 190 g ortho-Dichlorbenzol und 10 g Chinolin suspendiert und 5 Stunden am Rückfluß erhitzt. Der angefallene Feststoff wurde abgesaugt und mit Aceton gewaschen. Nach dem Trocknen wurden 22 g (99 % Ausbeute) des gewünschten Bisurethans mit einem Schmelzpunkt von 194°C bis 198°C erhalten; die spektroskoposchen Daten stimmten mit denen des Produkts von Beispiel 1 überein.

Stufe 2b); Beispiel 11.3:

Herstellung von 1-(4-Hydroxyphenylcarbamoyloxy)-10-hydroxydecan.

In einer Rührapparatur wurden 13,5 g (0,1 Mol als Monomer gerechnet) des nach Stufe 1) erhaltenen Polymers in einer Lösung von 26,1 g (0,15 Mol) 1,10-Decandiol, 190 g ortho-Dichlorbenzol und 10 g Chinolin 5 Stunden am Rückfluß erhitzt. Der angefallene Feststoff wurde abgesaugt und in 1 l Aceton aufgenommen. Ein geringer, unlöslicher Anteil wurde abgetrennt und verworfen. Die Acetonphase wurde am Rotationsverdampfer zur Trockne eingeengt, der Rückstand mit Methylenchlorid gewaschen und aus Toluol umkristallisiert. Als Kristallisat wurden 17,75 g (57 % Ausbeute) des gewünschten Dihydroxymonourethans erhalten. Es stimmte im Schmelzpunkt und den spektroskopischen Daten mit dem Produkt von Beispiel 8 überein. Ferner wurden 4,2 g eines in Toluol unlöslichen Rückstandes erhalten, welcher sich als das Dihydroxybisurethan erwies.

Herstellung von 1-(4-Hydroxyphenylcarbamoyloxy)-10-hydroxydecan als Eintopfreaktion; Beispiel 11.4:

In einer Rührapparatur mit 20 cm Vigreux-Kolonne und Destillationsaufsatz wurden 18,1 g (0,1 Mol) N-(4-Hydroxyphenyl)-ethylcarbamat, 190 g ortho-Dichlorbenzol und 10 g Chinolin zunächst 5 Stunden lang am Rückfluß erhitzt. Dann wurde erschöpfend Ethanol abdestilliert. Es wurden 26,1 g (0,15 Mol) 1,10-Decandiol hinzugefügt und nochmals 5 Stunden lang am Rückfluß erhitzt. Nach Aufarbeitung wie bei Beispiel 11.3 wurden 22 g (70 % Ausbeute) des gewünschten Dihydroxymono-urethans erhalten.

Beispiel 12

In einer Rührapparatur mit 20 cm Vigreux-Kolonne und Destillationsaufsatz wurden 10,1 g (0,05 Mol) 1,12-Dodecandiol in Gegenwart von 10 g Chinolin mit 29,3 g (0,1 Mol) N-(3,5-di-tert.-butyl-4-hydroxyphenyl)-1-propyl-carbamat in 190 g ortho-Dichlorbenzol unter erschöpfendem Abdestillieren von 1-Propanol umgesetzt. Dann wurde nochmals die gleiche Menge 1,12-Dodecandiol zugesetzt und 5 Stunden am Rückfluß erhitzt.

Die erkaltete Reaktionslösung wurde mit insgesamt 1 l 4 %iger Salzsäure gewaschen und die organische Phase durch Stehen über $Na_2CO_3$ getrocknet und entsäuert. Nach Entfernen des Lösungsmittels im Vakuum wurden 29,3 g (65 % Ausbeute) 1-(3,5-di-tert.-butyl-4-hydroxyphenylcarbamoyl-oxy)-12-hydroxydodecan als zähes Öl erhalten. Nach Umkristallisieren aus n-Hexan lag ein Feststoff mit einem Schmelzpunkt von 74-75°C vor.

Beispiel 13.1

Herstellung von Tris-(4-hydroxyphenylurethanen) aus Trihydroxyverbindungen:

In einer Rührapparatur mit 20 cm Vigreux-Kolonne und Destillationsaufsatz wurden 260 g ortho-Dichlorbenzol, 10 g Chinolin, 27,15 g (0,15 Mol) N-(4-hydroxyphenyl)-

ethylcarbamat und 15,3 g (0,05 Mol) eines Triols mit einer OH-Zahl von 550, einem Molekulargewicht von 306 und einem Mol-Verhältnis von Ethylenoxid zu Propylenoxid von 8:1 (statistische Verteilung), das durch Umsetzung von Ethylenoxid und Propylenoxid mit 2-Ethyl-2-hydroxymethyl-1,3-propandiol in bekannter Weise hergestellt worden war und bei Raumtemperatur flüssig war, unter erschöpfendem Abdestillieren von Ethanol zum Sieden erhitzt (ca. 5 Stunden). Das zum Teil zähflüssige Reaktionsgemisch wurde bei 120° C/20 mbar am Rotationsverdampfer von Lösungsmittel befreit. Der Rückstand wurde dreimal mit heißem Toluol (insgesamt 1 l) aufgekocht und dieses abgegossen.

Der Rückstand wurde am Rotationsverdampfer bei 120° C/ 5 mbar von Toluolresten befreit. Nach dem Abkühlen wurden 27,4 g vom gewünschten Tris-(4-hydroxyphenyl-urethan) erhalten. Weitere Details siehe Tabelle 5.

Beispiele 13.2 bis 13.4

In der gleichen Weise wurden weitere Triole zu den entsprechenden Tris-4-hydroxyphenyl-urethanen umgesetzt. Die Ergebnisse sind aus Tabelle 5 ersichtlich.

Beispiel 13.2:

Das eingesetzte Triol war hergestellt worden wie in Beispiel 13.1 beschrieben, es wies jedoch eine OH-Zahl von 250, ein Molekulargewicht von 673 und ein Mol-Verhältnis von Ethylenoxid zu Propylenoxid von 18,5:1 (statistische Verteilung) auf.

Beispiel 13.3:

Das eingesetzte Triol war hergestellt worden wie in Beispiel 13.1 beschrieben, jedoch nur durch Ethoxylierung, und wies eine OH-Zahl von 578,5 und ein Molekulargewicht von 291 auf.

Beispiel 13.4:

Das eingesetzte Triol war hergestellt worden wie in Beispiel 13.3 beschrieben. Es wies eine OH-Zahl von 282 und ein Molekulargewicht von 597 auf.

Tabelle 5

Reaktionsprodukt:

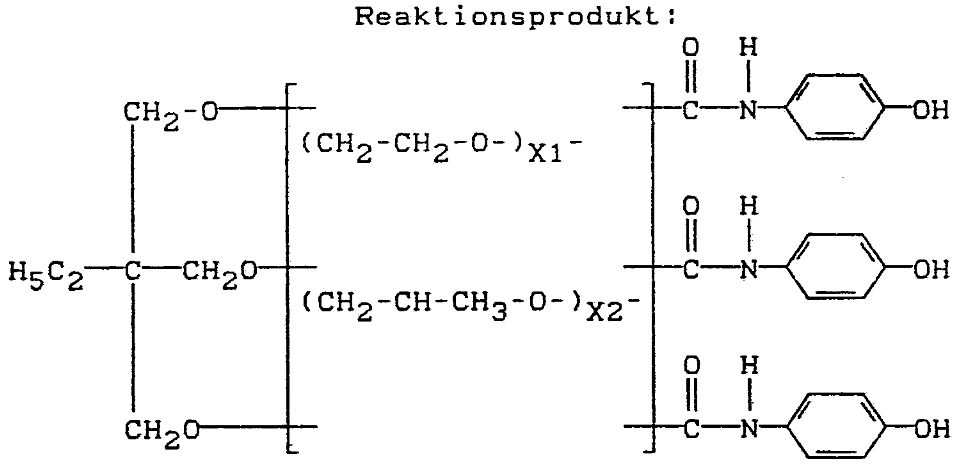

| Beispiel Nr. | $X_1$ | $X_2$ | Ausbeute % | Schmelzpunkt °C oder Konsistenz |
|---|---|---|---|---|
| 13.1 | 3,38 | 0,42 | 77 | 49-55 |
| 13.2 | 11,43 | 0,62 | 82 | zäh-viskos |
| 13.3 | 3,57 | 0 | 82 | zäh-viskos |
| 13.4 | 10,52 | 0 | 80 | zäh-viskos |

Beispiel 14

In einer Rührapparatur wurden 48,8 g (0,2 Mol) N,N'-Bis-(4-hydroxyphenyl)-harnstoff, 10,6 g (0,09 Mol) 1,6-Hexandiol, 3,4 g Di-n-butylzinndilaurat mit 500 ml Orthodichlorbenzol 16 Stunden am Rückfluß erhitzt. Aus dem erkalteten Reaktionsgemisch wurde der ausgefallene Feststoff abfiltriert, in 500 ml verdünnter Salzsäure aufgerührt, abgesaugt und mit Methanol nachgewaschen. Der Feststoff wurde in 500 ml 50° C warmem Aceton aufgenommen und die Lösung vom Unlöslichen abfiltriert. Aus der Acetonlösung wurden nach Eindampfen am Rotationsverdampfer 24,7 g des gleichen Bisurethans erhalten, wie in Beispiel 1.2 beschrieben. Die Ausbeute, bezogen auf das eingesetzte Diol, betrug 74 %.

Beispiel 15

Es wurde wie in Beispiel 14 verfahren, jedoch wurden 47,1 g (0,4 Mol) 1,6-Hexandiol eingesetzt. Als Reaktionsprodukt wurden 34,4 g des in Beispiel 8.1 beschriebenen Dihydroxymonourethans erhalten. Die Ausbeute, bezogen auf den eingesetzten Harnstoff, betrug 68 %.

Patentansprüche

1. Verfahren zur Herstellung von Hydroxyphenylurethanen der Formel (3)

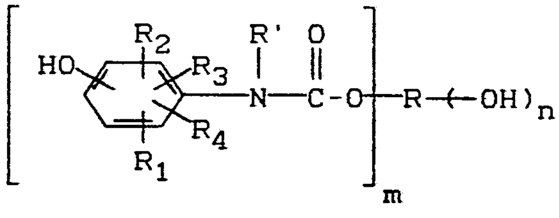

(3),

in der

m für eine ganze Zahl von 1 bis 4 und

n für Null oder eine ganze Zahl von 1 bis 3 steht und
die Summe von m + n mindestens 2 und höchstens 4 beträgt,

$R_1$ bis $R_4$ unabhängig voneinander Wasserstoff, Halogen, Cyano, Alkyl, Cycloalkyl, Aryl, Aralkyl, Oxyalkyl, Oxyphenyl oder Oxybenzyl,

R' Wasserstoff, Alkyl, Acyl oder Phenyl und

R im Falle von m + n von 2 bis 4

a) einen m + n-bindigen, geradkettigen, verzweigten oder cyclischen, gesättigten oder ungesättigten, gegebenenfalls substituierten Kohlenwasserstoffrest mit insgesamt 2 bis 30 C-Atomen oder

b) einen Rest der Formel (2.1)

$$Z\!-\!\left[\!\left(\!-OC_2H_3Y\!-\!\right)_r\right]_{m+n} \qquad (2.1)$$

mit Z = einem geradkettigen, verzweigten oder cyclischen, gesättigten oder ungesättigten, gegebenenfalls substituierten Rest mit insgesamt 3 bis 20 C-Atomen,

Y = H und/oder $CH_3$ (unabhängig von benachbarten Kettengliedern) und

r = einer ganzen Zahl von 1 bis 1000 oder einem gegebenenfalls nicht ganzzahligen Mittelwert von 1 bis 1000 und

im Falle von m + n = 2 auch

c) einen Rest der Formel (2.2)

$$-\left[-(-CH_2-)_s-O-\right]_t-(-CH_2-)_s- \qquad (2.2)$$

mit s = einer ganzen Zahl von 3 bis 6 und dem Produkt s · t = einer ganzen Zahl von 6 bis 3000 oder oder einem gegebenenfalls nicht ganzzahligen Mittelwert von 6 bis 3000 oder

d) einen Rest der Formel (2.3)

$$-(-C_2H_3Y-O-)_u-(-C_2H_3Y-)- \qquad (2.3)$$

mit u = einer ganzen Zahl von 2 bis 200 000 oder einem gegebenenfalls nicht ganzzahligen Mittelwert von 2 bis 200 000 und

Y in der gleichen Bedeutung wie bei Formel (2.1),

bedeuten,

dadurch gekennzeichnet, daß man Verbindungen mit mindestens einer harnstoff- oder urethanartig eingebundenen Struktureinheit der Formel (1)

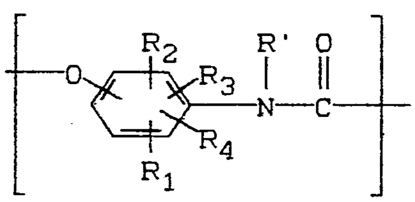

(1),

in der

$R_1$ bis $R_4$ und R' die bei Formel (3) angegebene Bedeutung haben,

mit Alkoholen der Formel (2)

$$(HO \leftarrow\!\!)_m R \leftarrow OH)_n \qquad (2),$$

in der

m, n und R die bei Formel (3) angegebene Bedeutung haben, bei erhöhter Temperatur und gegebenenfalls in Gegenwart von Lösungsmitteln und/oder Katalysatoren umsetzt.

2. Verfahren nach Anspruch 1, dadurch gekennzeichnet, daß man als Verbindung der Formel (1) ein Polyurethan der Formel (4)

$$-\!(\!-1-\!)_q\!- \qquad (4),$$

in der

1 für eine Struktureinheit der Formel (1) und

q für 500 bis $10^6$ dividiert durch das Molekulargewicht der jeweiligen Struktureinheit (1)

stehen,

ein Hydroxyphenylurethan der Formel (5)

$$H-(-1-)-OR_5 \qquad (5),$$

in der

1 für eine Struktureinheit der Formel (1) und

$R_5$ für gegebenenfalls substituiertes $C_1$-$C_5$-Alkyl, Phenyl oder Benzyl

stehen,

ein Hydroxyphenylurethan der Formel (6)

$$[H-(-1-)-O]_{m'}-R \qquad (6),$$

in der

1 für eine Struktureinheit der Formel (1) und

m' für eine ganze Zahl von 2 bis 4 stehen und

R die bei Formel (3) angegebene Bedeutung hat,

oder
einen symmetrisch substituierten Harnstoff der Formel (7)

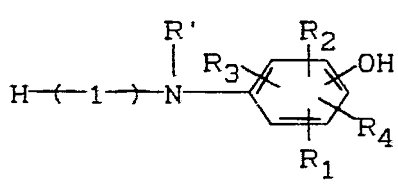

(7),

in der

1 für eine Struktureinheit der Formel (1) und

$R_1$ bis $R_4$ und R' die bei Formel (3) angegebene Bedeutung haben,

einsetzt.

3) Verfahren nach Ansprüchen 1 und 2, dadurch gekennzeichnet, daß man es bei Temperaturen im Bereich 80 bis 250° C durchführt.

4) Verfahren nach Ansprüchen 1 bis 3, dadurch gekennzeichnet, daß man es bei Drucken im Bereich 10 mbar bis 20 bar durchführt.

5) Verfahren nach Ansprüchen 1 bis 4, dadurch gekennzeichnet, daß man in Gegenwart eines Lösungsmittels arbeitet, das zumindest den einzusetzenden Alkohol der Formel (2) bei Reaktionstemperatur zu lösen vermag.

6) Verfahren nach Ansprüchen 1 bis 5, dadurch gekennzeichnet, daß man es in Gegenwart von Katalysatoren durchführt.

7) Verfahren nach Anspruch 6, dadurch gekennzeichnet, daß man es in Gegenwart von Lewis-Säuren, Brönsted-Säuren, Lewis-Basen, Brönsted-Basen und/oder Gemischen von Säuren und Basen durchführt.

8) Verfahren nach Anspruch 7, dadurch gekennzeichnet, daß man es in Gegenwart von tertiären Aminen, tertiären Phosphinen oder metallorganischen Verbindungen der Metalle Lithium, Natrium, Kalium, Magnesium, Kalcium, Aluminium, Thallium, Titan oder Zinn durchführt.

9) Verfahren nach Ansprüchen 1 bis 8, dadurch gekennzeichnet, daß man die Reaktanten im stöchiometrischen Verhältnis oder in einem bis zu ± 20 % davon abweichenden Verhältnis einsetzt.

10) Verfahren nach Ansprüchen 1 bis 8, dadurch gekennzeichnet, daß man im Falle der Herstellung von Hydroxyphenylurethanen der Formel (3) mit m = 1 den Alkohol der Formel (2) im großen Überschuß einsetzt.